(19) Europäisches Patentamt
European Patent Office
Office européen des brevets

(11) Veröffentlichungsnummer: **0 419 963 B1**

# (12) EUROPÄISCHE PATENTSCHRIFT

(45) Veröffentlichungstag der Patentschrift: **07.12.94**

(51) Int. Cl.[5]: **C07D 487/04**, A01N 43/90, //(C07D487/04,239:00,235:00), (C07D487/04,239:00,239:00)

(21) Anmeldenummer: **90117702.2**

(22) Anmeldetag: **14.09.90**

Die Akte enthält technische Angaben, die nach dem Eingang der Anmeldung eingereicht wurden und die nicht in dieser Patentschrift enthalten sind.

## (54) 1,2,3,4-Tetrahydro-5-nitro-pyrimidin-Derivate.

(30) Priorität: **27.09.89 DE 3932167**

(43) Veröffentlichungstag der Anmeldung: **03.04.91 Patentblatt 91/14**

(45) Bekanntmachung des Hinweises auf die Patenterteilung: **07.12.94 Patentblatt 94/49**

(84) Benannte Vertragsstaaten: **BE CH DE FR GB IT LI NL**

(56) Entgegenhaltungen:
**EP-A- 0 247 477**
**EP-A- 0 316 843**
**EP-A- 0 316 845**

(73) Patentinhaber: **BAYER AG**

**D-51368 Leverkusen (DE)**

(72) Erfinder: **Gesing, Ernst R., Dr.**
**Trillser Graben 4**
**D-4006 Erkrath-Hochdahl (DE)**
Erfinder: **Becker, Benedikt, Dr.**
**Versuchsstation für Pflanzenschutz, Steinmannhof**
**I-39053 Pineta De Laives (IT)**
Erfinder: **Hartwig, Jürgen, Dr.**
**Franz-Esser-Strasse 44**
**D-5090 Leverkusen 3 (DE)**
Erfinder: **Stendel, Wilhelm, Dr.**
**In den Birken 55**
**D-5600 Wuppertal 1 (DE)**

EP 0 419 963 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 1,2,3,4-Tetrahydro-5-nitro-pyrimidin-Derivate, ein Verfahren zu ihrer Herstellung und ihre Verwendung in Schädlingsbekämpfungsmitteln, insbesondere als Insektizide. Darüber hinaus besitzen die neuen Verbindungen eine stark ausgeprägte ektoparasitizide Wirksamkeit.

Es ist bereits bekannt, daß bestimmte Pyrimidino-thiazine wie z. B. 7-Ethyl-9-nitro-3,4,7,8-tetrahydro-(2H,6H)-pyrimidino-[4,3-b]-1,3-thiazin insektizide Eigenschaften aufweisen (vergl. US-PS 4 031 087).

Weiterhin ist bekannt, daß bestimmte nitrierte Stickstoffheterocyclen insektizide Eigenschaften besitzen (vergleiche EP 0 316 845).

Es wurden nun die neuen 1,2,3,4-Tetrahydro-5-nitro-pyrimidin-Derivate der Formel (I),

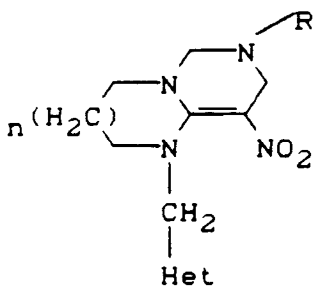

(I)

in welcher

n für die Zahl 0 oder 1 steht,

Het für gegebenenfalls substituiertes Pyridyl oder gegebenenfalls substituiertes Thiazolyl steht und

R für Monohalogen-tert-Butyl, für unsubstituiertes Cycloalkyl mit 9 bis 18 Kohlenstoffatomen, für Cycloalkyl mit 7 bis 18 Kohlenstoffatomen an welches 1 oder 2 Carbocyclen anelliert sind, für einfach oder mehrfach substituiertes Cyclohexyl welches substituiert ist durch: Alkoxy, Mono- oder Dialkylamino, Cycloalkylamino, Mono- oder Dialkylaminocarbonyl, Cycloalkyl, Cycloalkylalkyl und/oder durch Phenylalkyl, für Cyclohexyl, an welches 1 oder 2 weitere Carbocyclen anelliert sind, für Cycloalkylalkyl mit 3 bis 10 Kohlenstoffatomen im Cycloalkyl- und 1 bis 5 Kohlenstoffatomen im Alkylteil, welches im Cycloalkylteil einfach oder mehrfach substituiert ist, für gegebenenfalls einfach oder mehrfach substituiertes Heterocyclyl mit 2 bis 8 Kohlenstoffatomen und 1 bis 4 Heteroatomen (vorzugsweise Stickstoff, Sauerstoff und/oder Schwefel) oder Heterogruppierungen (vorzugsweise $SO_2$), an welches gegebenenfalls 1 oder 2 Carbocyclen anelliert sind, für gegebenenfalls einfach oder mehrfach substituiertes Tetrahydrofurylalkyl mit 1 bis 6 Kohlenstoffatomen im Alkylteil oder für gegebenenfalls einfach oder mehrfach substituiertes Piperidylalkyl mit 1 bis 6 Kohlenstoffatomen im Alkylteil, für Adamantyl, Adamantylmethyl oder für eine der folgenden Gruppierungen (A), (B) und (C), welche gegebenenfalls substituiert sind:

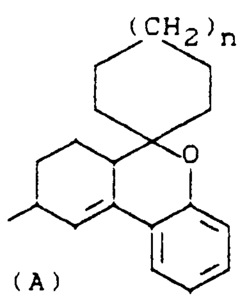

,

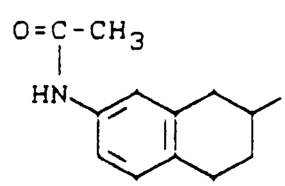

,

(A)

(n steht für 0 oder 1)

(B)

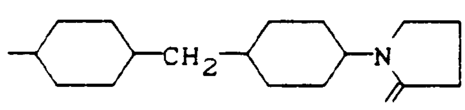

(C)

und deren Säureadditions-Salze gefunden.

Einige der neuen 1,2,3,4-Tetrahydro-5-nitro-pyrimidin-Derivate der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man die 1,2,3,4-Tetrahydro-5-nitro-pyrimidin-Derivate der Formel (I) und deren Säureadditions-Salze erhält, wenn man Nitromethylen-Derivate der Formel (II),

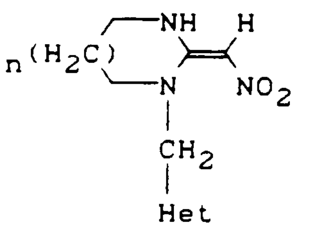

(II)

in welcher

n und Het die oben angegebenen Bedeutungen haben,

mit Aminen der Formel (III),

$R\text{-}NH_2$ (III)

in welcher

R die oben angegebene Bedeutung hat,

in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen Säuren addiert.

Monohalogen-tert-Butyl in der Definition von R bedeutet vorzugsweise Fluor, Chlor- oder Brom-, insbesondere Fluor-tert-Butyl.

Carbocyclen, welche an andere Ringe anelliert sein können, sind gesättigt oder ungesättigt oder aromatisch. Sie bilden mit den Kohlenstoffatomen, an die sie anelliert sind, vorzugsweise Cyclohexyl oder Phenylringe. Die Carbocyclen können einen oder mehrere, gleiche oder verschiedene der weiter unten aufgeführten Substituenten tragen. Vorzugsweise sind sie jedoch unsubstituiert.

Heterocyclyl in der Definition von R bedeutet in den allgemeinen Formeln vorzugsweise heteroparaffinische, heteroaromatische und heteroolef inische 5- bis 7-gliedrige Ringe mit vorzugsweise 1 bis 3, insbesondere 1 oder 2 gleichen oder verschiedenen Heteroatomen oder Heterogruppen. Als Heteroatome stehen Sauerstoff, Schwefel oder Stickstoff und als Heterogruppen $SO_2$ oder N-($C_1$-$C_4$-Alkyl). Vorzugsweise seien Pyrrolidinyl, Piperidinyl, Furyl, Thiophenyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl und 1,2,4-Diazepinyl genannt.

Die gegebenenfalls substituierten Reste in den allgemeinen Formeln können einen oder mehrere, vorzugsweise 1 bis 3, insbesondere 1 oder 2 gleiche oder verschiedene Substituenten tragen. Als Substituenten seien vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Cycloalkyl, Cycloalkylamino oder Cycloalkylalkyl mit vorzugsweise 3 bis 7, insbesondere 5 oder 6 Kohlenstoffatomen im Cycloalkylteil und vorzugsweise 1 bis 5, insbesondere 1 bis 4 Kohlenstoffatome im geradkettigen oder verzweigten Alkylteil; Aralkyl, vorzugsweise Phenylalkyl mit vorzugsweise 1 bis 4, insbesondere 1 bis 3 Kohlenstoffatomen im Alkylteil; Alkoxy mit vorzugsweise 1 bis 4, insbesondere 1 oder 2 Kohlenstoffatomen, wie Methoxy, Ethoxy, n- und i-Propoxy und n-, i- und t-Butoxy, Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino oder Mono- und Dialkylaminocarbonyl mit vorzugsweise 1 bis 4, insbesondere 1 oder 2 Kohlenstoffatomen je Alkylgruppe, wie

Methylamino, Methyl-ethyl-amino, n- und i-Propyl-amino, und Methyl-n-butylamino; Carboxy; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl.

Die Pyridyl- und Thiazolyl-Reste in der Definition von Het tragen vorzugsweise 1 oder 2 gleiche oder verschiedene Substituenten, wobei als Substituenten vorzugsweise Halogen (Fluor, Chlor, Brom und Jod, vorzugsweise Fluor oder Chlor, insbesondere Chlor) genannt sei. Besonders bevorzugt steht Het für 2-Chlor-1,3-thiazol-5-yl und 2-Chlorpyridin-5-yl.

Die hier aufgeführten bevorzugten Definitionen gelten auch für die im folgenden aufgeführten bevorzugten Kombinationen der Definitionen in entsprechender Weise.

Überraschenderweise zeichnen sich die erfindungsgemäßen 1,2,3,4-Tetrahydro-5-nitro-pyrimidin-Derivate der Formel (I) in überragender Weise durch eine hohe Wirksamkeit als Insektizide und Ektoparasitizide aus.

Die Erfindung betrifft vorzugsweise Verbindungen der Formel (I), in welcher

- n für die Zahlen 0 oder 1 steht,
- Het für gegebenenfalls durch Halogen substituiertes Pyridyl oder für gegebenenfalls durch Halogen substituiertes Thiazolyl steht und
- R für durch Fluor oder Chlor monosubstituiertes tert-Butyl, für unsubstituiertes Cycloalkyl mit 9 bis 16 Kohlenstoffatomen, für Cycloalkyl mit 7 bis 16 Kohlenstoffatomen, an welches 1 oder 2 6-gliedrige aromatische oder gesättigte Carbocyclen anelliert sind, für einfach bis dreifach substituiertes Cyclohexyl, welches substituiert ist durch: Alkoxy mit 1 bis 4 Kohlenstoffatomen, Mono- oder Dialkylamino mit jeweils 1 bis 4 Kohlenstoffatomen in den einzelnen Alkylteilen, Cycloalkylamino mit 3 bis 7 Kohlenstoffatomen, Mono- oder Dialkylaminocarbonyl mit jeweils 1 bis 4 Kohlenstoffatomen in den einzelnen Alkylteilen, Cycloalkyl mit 3 bis 7 Kohlenstoffatomen, Cycloalkylalkyl mit 3 bis 7 Kohlenstoffatomen im Cycloalkyl- und 1 bis 5 Kohlenstoffatomen im Alkylteil und/oder durch Phenylalkyl mit 1 bis 4 Kohlenstoffatomen im Alkylteil, für Cyclohexyl, an welches 1 oder 2 6-gliedrige aromatische oder gesättigte Carbocyclen anelliert sind, für Cycloalkylalkyl mit 3 bis 7 Kohlenstoffatomen im Cycloalkyl- und 1 bis 4 Kohlenstoffatomen im Alkylteil, welches im Cycloalkylteil einfach bis dreifach durch Alkyl oder Halogenalkyl mit jeweils 1 bis 4 Kohlenstoffatomen und gegebenenfalls 1 bis 9 Fluor- und/oder Chloratomen substituiert ist, für gegebenenfalls einfach oder mehrfach durch Alkyl und/oder Halogenalkyl mit jeweils 1 bis 4 Kohlenstoffatomen und gegebenenfalls 1 bis 9 Fluor-und/oder Chloratomen substituiertes Heterocyclyl mit 3 bis 6 Kohlenstoffatomen und 1 bis 3 Heteroatomen oder Heterogruppierungen aus der Reihe Sauerstoff, Schwefel, Stickstoff, $SO_2$, an welches gegebenenfalls 1 oder 2 6-gliedrige aromatische oder gesättigte Carbocyclen anelliert sind, für gegebenenfalls einfach bis dreifach durch Alkyl und/oder Halogenalkyl mit jeweils 1 bis 4 Kohlenstoffatomen und gegebenenfalls 1 bis 9 Fluor-und/oder Chloratomen substituiertes Tetrahydrofurylalkyl mit 1 bis 4 Kohlenstoffatomen im Alkylteil oder für gegebenenfalls einfach bis dreifach durch Alkyl und/oder Halogenalkyl mit jeweils 1 bis 4 Kohlenstoffatomen und gegebenenfalls 1 bis 9 Fluor- und/oder Chloratomen substituiertes Piperidylalkyl mit 1 bis 4 Kohlenstoffatomen im Alkylteil oder für eine der folgenden Gruppierungen (A) bis (F) steht, welche gegebenenfalls durch Alkyl mit 1 bis 4 Kohlenstoffatomen substituiert sind:

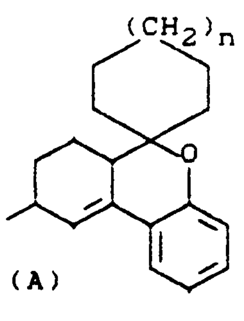

(A)

,

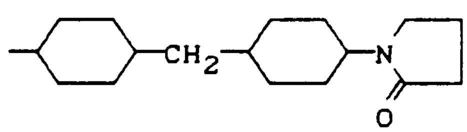

(C)

,

(n steht für 0 oder 1)

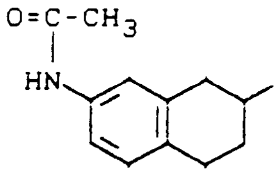

(B)

,

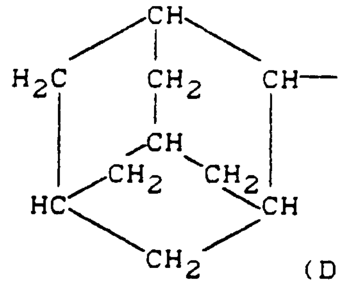

(D)

,

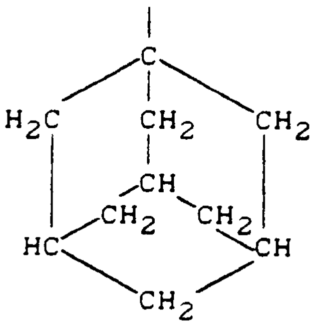

(E)

,

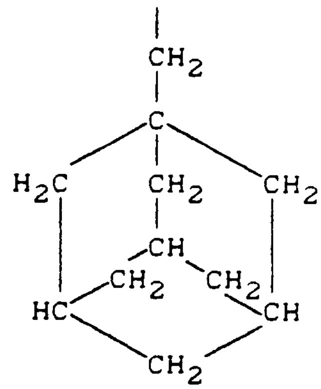

(F)

Besonders bevorzugt sind diejenigen Verbindungen der Formel (I), in welcher

n für die Zahl 0 oder 1 steht,

Het für

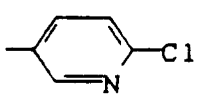 oder 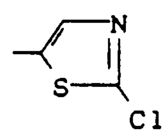

steht und

R für Fluor-tert-Butyl, für unsubstituiertes Cycloalkyl mit 9 bis 12 Kohlenstoffatomen, für Cycloalkyl mit 7 bis 12 Kohlenstoffatomen, an welches 1 oder 2 6-gliedrige aromatische Carbocyclen anelliert sind, für 1- oder 2fach substituiertes Cyclohexyl, welches substituiert ist durch:

Methoxy, Ethoxy, n- oder i-Propoxy, Methylamino, Dimethylamino, Ethylamino, Diethylamino, Ethylmethylamino, n-Propylamino, Di-(n-propyl)amino, Methyl(n-propyl)amino, Ethyl(n-propyl)amino, Isopropylamino, Di-(isopropyl)amino, Methylisopropylamino, Ethylisopropylamino, Isopropyl(n-propyl)amino oder Cyclopentylamino, Cyclohexylamino, Methylaminocarbonyl, Dimethylaminocarbonyl, Ethylaminocarbonyl, Diethylaminocarbonyl, Ethylmethylaminocarbonyl, n-Propylaminocarbonyl, Di(n-propyl)aminocarbonyl, Methyl(n-propyl)-aminocarbonyl, Ethyl(n-propyl)aminocarbonyl, Isopropylaminocarbonyl, Di(isopropyl)aminocarbonyl, Methylisopropylaminocarbonyl, Ethylisopropylaminocarbonyl, Isopropyl(n-propyl)aminocarbonyl, Cyclopentyl, Cyclohexyl, Cyclopentlymethyl, Cyclopentylethyl, Cyclopentyl-n-propyl, Cyclopentylisopropyl, Cyclohexylmethyl, Cyclohexylethyl, Cyclohexyl-n-propyl, Cyclohexylisopropyl, Phenylmethyl, Phenylethyl, Phenyl-n-propyl und/oder Phenylisopropyl, für Cyclohexyl an welches 1 oder 2 6-gliedrige aromatische oder gesättigte Carbocyclen anelliert sind, für Cyclopentylmethyl, Cyclopentylethyl, Cyclohexylmethyl oder Cyclohexylethyl, wobei diese Reste im Cycloalkylteil 1- oder 2-fach durch Methyl und/oder Trifluormethyl substituiert sind, für gegebenenfalls einfach bis sechsfach durch Methyl und/oder Trifluormethyl substituiertes Heterocyclyl mit 5 bis 12 Kohlenstoffatomen und 1 oder 2 Heteroatomen oder Heterogruppierungen aus der Reihe Sauerstoff, Schwefel, Stickstoff oder $SO_2$, an welches gegebenenfalls 1 oder 2 6-gliedrige aromatische Carbocyclen anelliert sind, für Tetrahydrofurylmethyl oder Tetrahydrofurylethyl, für Piperidylmethyl, Piperidylethyl oder Piperidyl-n-propyl oder für eine der folgenden Gruppierungen (A) bis (F):

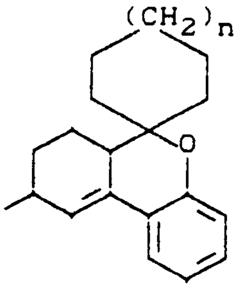,

(n steht für 0 oder 1)

(A)

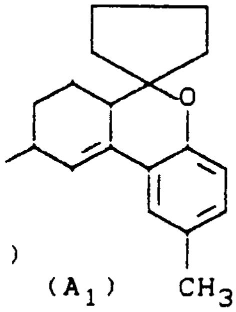,

($A_1$)

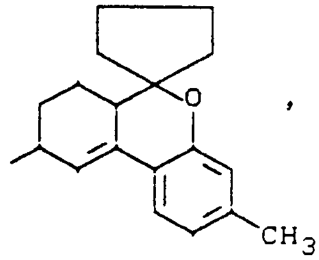,

($A_2$)

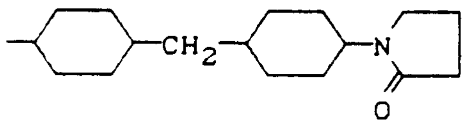,

(C)

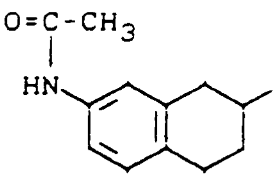,

(B)

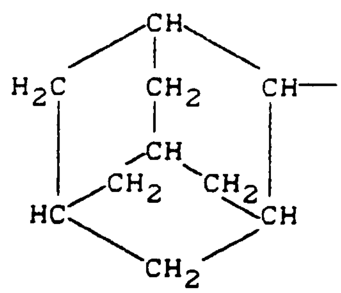,

(D)

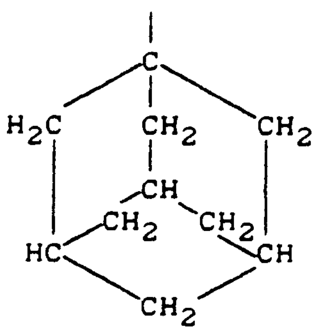,

(E)

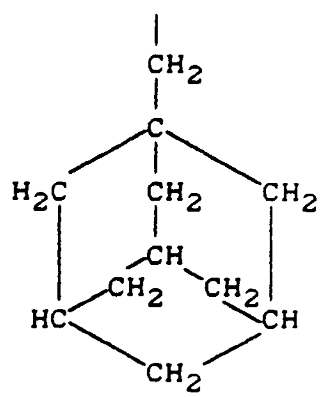

(F)

Die für die Verbindung der allgemeinen Formel (I) angegebenen allgemeinen und bevorzugten Restedefinitionen gelten auch für die Verbindungen der übrigen allgemeinen Formeln (Zwischen- bzw. Vorprodukte) entsprechend.

Bevorzugte erfindungsgemäße Verbindungen sind auch Additionsprodukte aus Säuren und den 1,2,3,4-Tetrahydro-5-nitro-pyrimidin-Derivaten der Formel (I).

Zu den Säuren, die addiert werden können, gehören vorzugsweise Halogenwasserstoffsäuren, wie z B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Verwendet man beispielsweise für das erfindungsgemäße Verfahren 3-(2-Chlor-pyridin-5-yl-methyl)-2-nitromethylen-imidazolidin, 4-(1-Methyl-1-Cyclohexylethyl)cyclohexylamin und mindestens die zweifache molare Menge Formaldehyd als Ausgangsstoffe, so kann die entsprechende Reaktion durch das folgende Formelschema wiedergegeben werden:

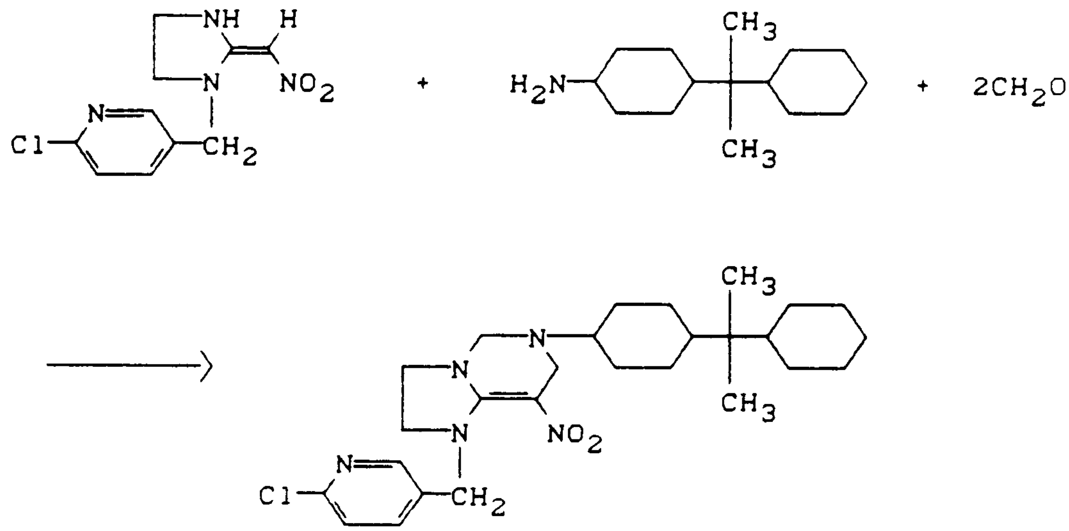

Die beim erfindungsgemäßen Verfahren als Ausgangsstoffe zu verwendenden Verbindungen der Formel (II) sind bekannt und/oder lassen sich nach bekannten Methoden herstellen (vergl. z. B. DE-OS 2 514 402, EP-OS 136 636, EP-OS 154 178 und EP-OS 163 855).

Die beim erfindungsgemäßen Verfahren außerdem als Ausgangsstoffe zu verwendenden Amine der Formel (III) sind allgemein bekannte Verbindungen der organischen Chemie.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesetzt.

Das erfindungsgemäße Verfahren wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zwischen -20 °C und +120 °C, vorzugsweise bei Temperaturen zwischen 0 °C und +80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingesetzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter

Weise, z. B. durch Abfiltrieren, isoliert und gegebenenfalls durch Waschen mit einem inerten organischen Lösungsmittel gereinigt werden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forf icula auricularia.

Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Acanthoscelides obtectus, Hylotrupes bajulus, Agelastica alni, Leptinotarsa decemlineata, Phaedon cochleariae Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp..

Aus der Ordnung der Arachnida z. B. Scorpio maurus, Latrodectus mactans.

Die erfindungsgemäßen Wirkstoffe der Formel (I) zeichnen sich durch eine hervorragende insektizide Wirksamkeit aus. Sie zeigen insbesondere beim Einsatz als Blattinsektizide und Bodeninsektizide eine hervorragende Wirkung gegen Maden wie z. B. Phorbia antiqua-Maden, gegen Käferlarven, wie z. B. Phaedon cochleariae und Blattläuse, wie z. B. Aphis fabae.

Weiterhin zeigen die erfindungsgemäßen Wirkstoffe der Formel (I) eine hervorragende Wurzelsystemische Wirkung z. B. gegen Phaedon cochleariae-Larven und Blattläuse wie Myzus persicae und eignen sich darüber hinaus auch ausgezeichnet zur Saatgutbehandlung z. B. gegen Maden wie Phorbia antiqua, gegen Käferlarven wie Phaedon cochleariae und Blattläuse wie Aphis fabae.

Die erfindungsgemäßen Wirkstoffe eignen sich außerdem hervorragend zur Bekämpfung von Ektoparasiten wie z. B. Lucilia cuprina-Larven, Blattella germanica und Sitophilus granarius.

Die neuen Verbindungen sind also besonders gut für einen Einsatz zur Bekämpfung von Blattinsekten und Bodeninsekten sowie zur Saatgutbehandlung und Bekämpfung von Ektoparasiten geeignet. Besonders bevorzugt werden die Verbindungen als Pflanzenschutzmittel eingesetzt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von lästigen Insekten, Milben, Zecken usw. auf dem Gebiet der Tierhaltung und Viehzucht, wobei durch die Bekämpfung der Schädlinge bessere Ergebnisse, z. B. höhere Milchleistungen, höheres Gewicht, schöneres Tierfell usw. erreicht werden können.

Sie zeigen insbesondere beim Einsatz als Ektoparasitizide eine ausgezeichnete Wirkung gegen Blowfly-larven wie z. B. Lucilia cuprina.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns der Tiere oder durch die entsprechende Behandlung der Umgebung der Tiere (z.B. Tierställe).

Die Herstellung sowie die biologische Wirksamkeit der erfindungsgemäßen Verbindungen soll anhand der folgenden Beispiele erläutert werden (in den Herstellungsbeispielen bedeutet Raumtemperatur: 18 bis 20 °C):

Herstellungsbeispiele

Beispiel 1

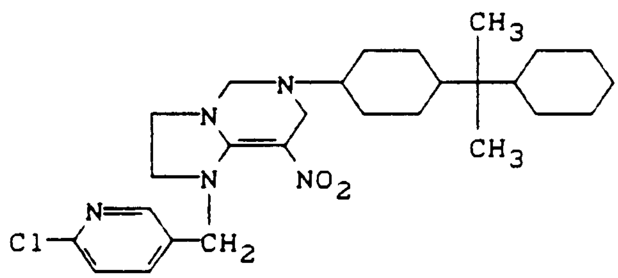

Zu einer Mischung aus 2,54 g (0,01 Mol) 3-(2-Chlorpyridin-5-yl-methyl)-2-nitromethylen-imidazolidin und 2,23 g (0,01 Mol) 4-(1-Methyl-1-Cyclohexylethyl)cyclohexylamin in 90 ml Ethanol werden bei Raumtemperatur 1,5 ml (0,02 Mol) 37 % wäßrige Formaldehydlösung zugetropft und 3 Stunden unter Rückfluß erhitzt. Nach dem Abkühlen auf Raumtemperatur wird das Lösungsmittel im Vakuum entfernt, der Rückstand mit Ether versetzt und abgesaugt.

Man erhält 3,5 g (70 % der Theorie) 6,7-Dihydro-6-[4-(1-Methyl-1-Cyclohexylethyl)-cyclohexyl]-8-nitro-(5H)-3-(2-chlorpyridin-5-yl-methyl)-imidazolidino-[2,3-f]-pyrimidin vom Schmelzpunkt 115 °C (Zers.).

Analog Beispiel 1 bzw. dem erfindungsgemäßen Verfahren können die in der nachfolgenden Tabelle 1 angegebenen Verbindungen der Formel (I) hergestellt werden:

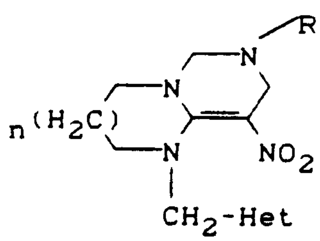

(I)

Tabelle 1

| Bsp. Nr. | n | R | Het | Schmelzpunkt |
|---|---|---|---|---|
| 2 | 0 | 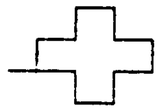 | 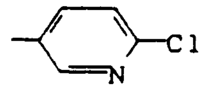 | 175-179° C |
| 3 | 1 | 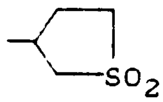 | 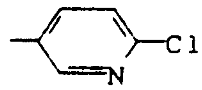 | Öl |
| 4 | 1 | 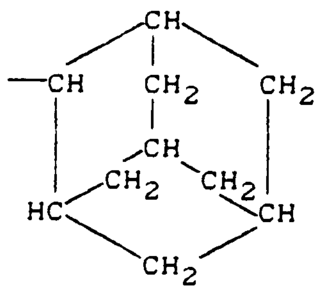 | 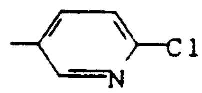 | 156° C |
| 5 | 0 | 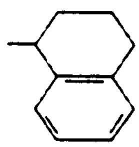 | 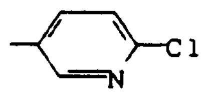 | 164-165° C |
| 6 | 1 | 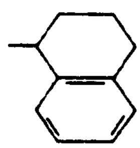 | 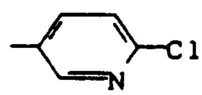 | 172-175° C |
| 7 | 1 | 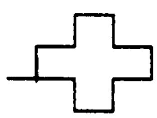 | 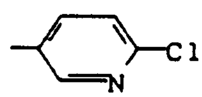 | 158-164° C |

Tabelle 1 (Fortsetzung)

| Bsp. Nr. | n | R | Het | Schmelzpunkt |
|---|---|---|---|---|
| 8 | 0 | 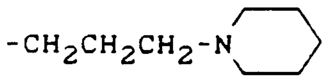 | 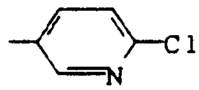 | 103-112° C |
| 9 | 0 | 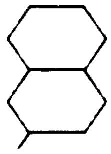 | 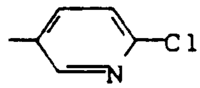 | 144° C (Zers.) |
| 10 | 1 | 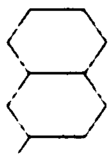 | 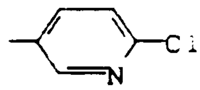 | 114-120° C |
| 11 | 0 | 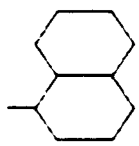 | 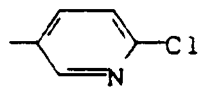 | 119-123° C (Zers.) |
| 12 | 1 | 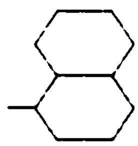 | 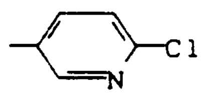 | 134° C (Zers.) |
| 13 | 0 | 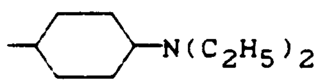 | 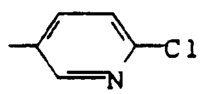 | 140-148° C |
| 14 | 1 | 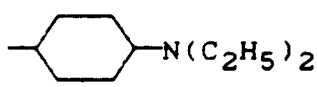 | 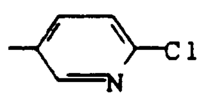 | 148° C (Zers.) |
| 15 | 0 | 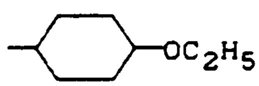 | 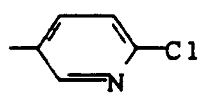 | 127-134° C |
| 16 | 1 | 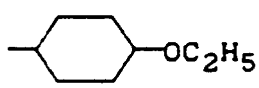 | 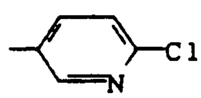 | 153° C (Zers.) |

Tabelle 1 (Fortsetzung)

| Bsp. Nr. | n | R | Het | Schmelzpunkt |
|---|---|---|---|---|
| 17 | 0 | 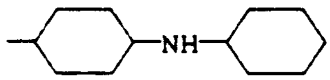 | 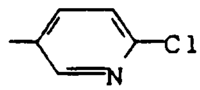 | 146-150° C |
| 18 | 1 | 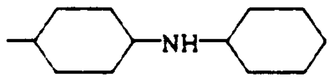 | 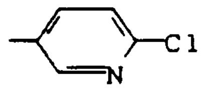 | 139-141° C |
| 19 | 0 | 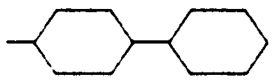 | 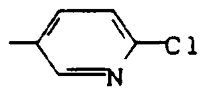 | 173° C (Zers.) |
| 20 | 1 | 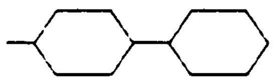 | 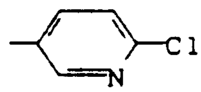 | 159° C (Zers.) |
| 21 | 1 | 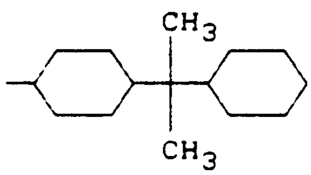 | 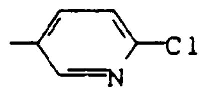 | 139-142° C |
| 22 | 0 | 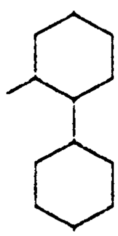 | 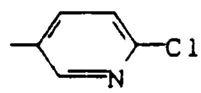 | 98° C |
| 23 | 0 | 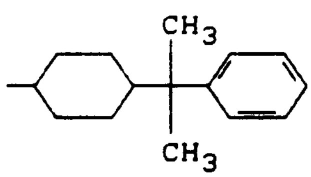 | 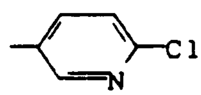 | 158° C |
| 24 | 0 | 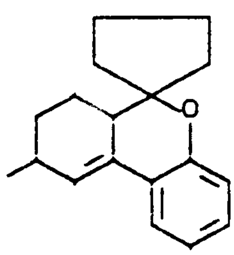 | 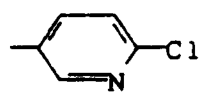 | 190° C (Zers.) |

Tabelle 1 (Fortsetzung)

| Bsp. Nr. | n | R | Het | Schmelzpunkt |
|---|---|---|---|---|
| 25 | 0 | 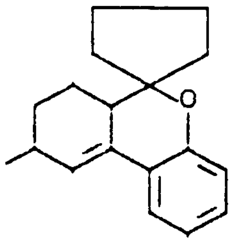 Isomeres | 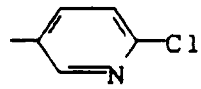 | 91-101° C (Zers.) |
| 26 | 0 | 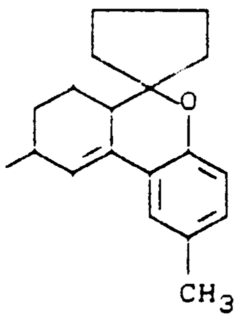 | 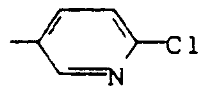 | 111° C |
| 27 | 0 | 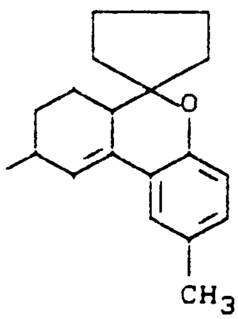 Isomeres | 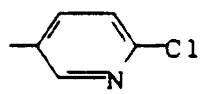 | 145° C (Zers.) |
| 28 | 0 | 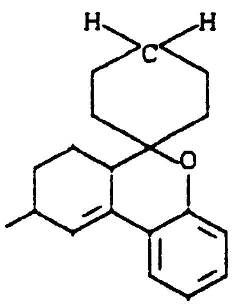 | 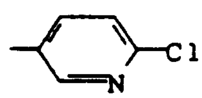 | 130-135° C (Zers.) |

Tabelle 1 (Fortsetzung)

| Bsp. Nr. | n | R | | Het | Schmelzpunkt |
|---|---|---|---|---|---|
| 29 | 0 | 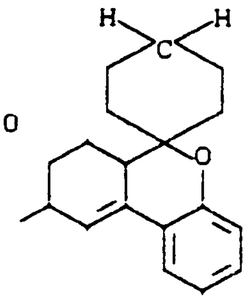 | Isomeres | 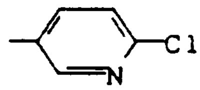 | 179° C (Zers.) |
| 30 | 0 | 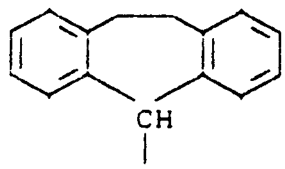 | | 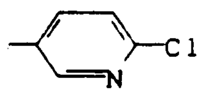 | 160-174° C (Zers.) |
| 31 | 1 | 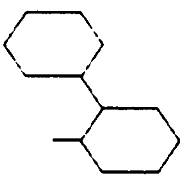 | | 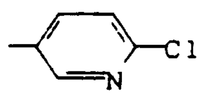 | 72° C |
| 32 | 1 | 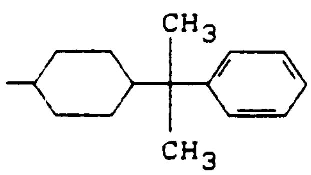 | | 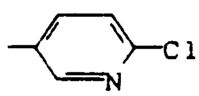 | 165° C (Zers.) |
| 33 | 1 | 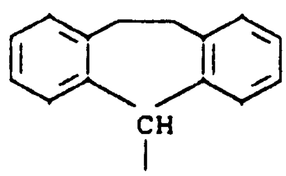 | | 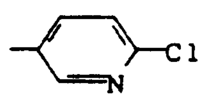 | 168° C (Zers.) |
| 34 | 0 | 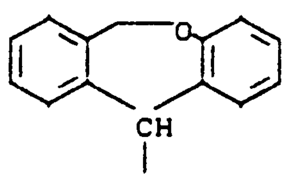 | | 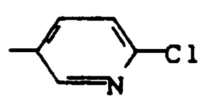 | 168° C (Zers.) |

Tabelle 1 (Fortsetzung)

| Bsp. Nr. | n | R | Het | Schmelzpunkt |
|---|---|---|---|---|
| 35 | 0 | 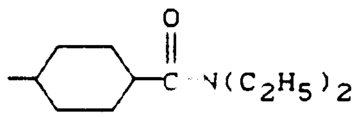 | 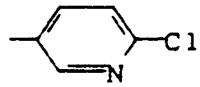 | 122-132° C (Zers.) |
| 36 | 1 | 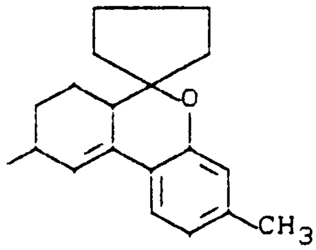 | 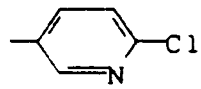 | 115° C |
| 37 | 0 | 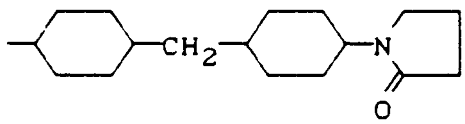 | 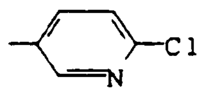 | 149° C |
| 38 | 0 | 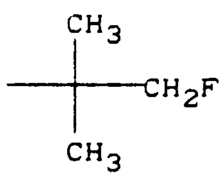 | 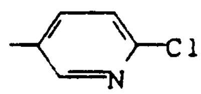 | 167° C |
| 39 | 0 | 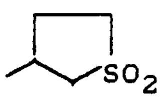 | 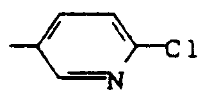 | 87-90° C (Zers.) |
| 40 | 0 | 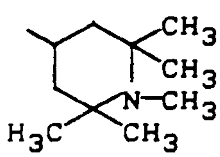 | 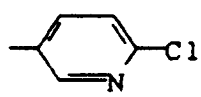 | 182° C (Zers.) |
| 41 | 1 | 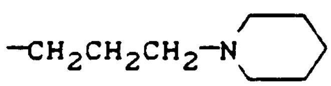 | 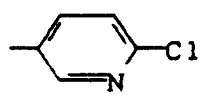 | 123-125° C |

Tabelle 1 (Fortsetzung)

| Bsp. Nr. | n | R | Het | Schmelzpunkt |
|---|---|---|---|---|
| 42 | 1 | 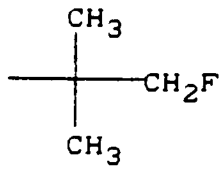 | 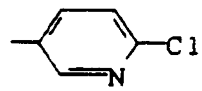 | |
| 43 | 0 | 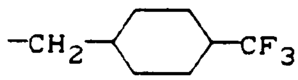 | 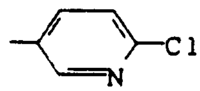 | 112° C |
| 44 | 1 | 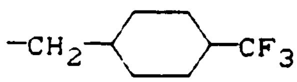 | 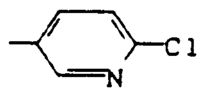 | 74-76° C |
| 45 | 0 | 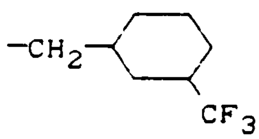 | 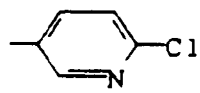 | 129° C |
| 46 | 1 | 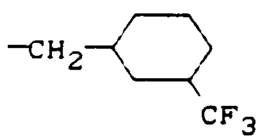 | 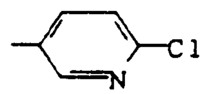 | 126° C |
| 47 | 1 | 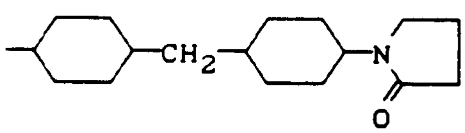 | 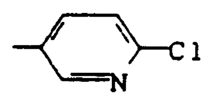 | 130° C |
| 48 | 1 | 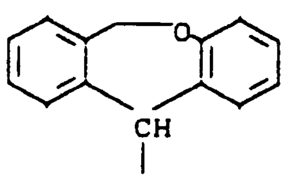 | 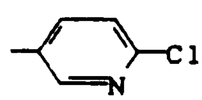 | 185° C |

Tabelle 1 (Fortsetzung)

| Bsp. Nr. | n | R | Het | Schmelzpunkt |
|---|---|---|---|---|
| 49 | 0 | 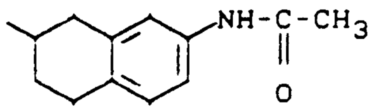 | 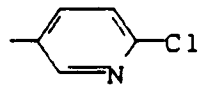 | 212° C |
| 50 | 1 | 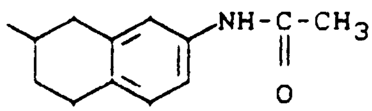 | 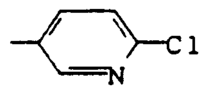 | 165° C |
| 51 | 0 | 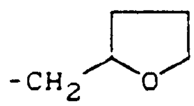 | 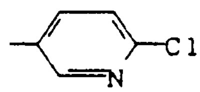 | 138° C |
| 52 | 1 | 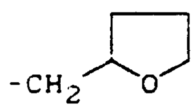 | 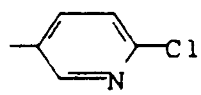 | 101° C |
| 53 | 0 | 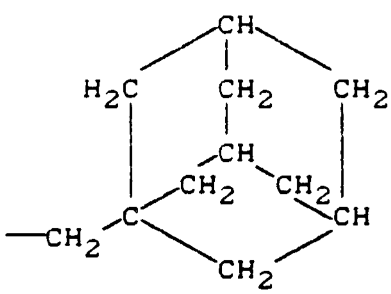 | 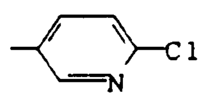 | 181° C |
| 54 | 1 | 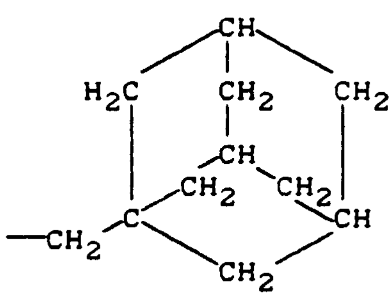 | 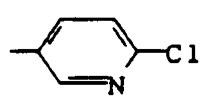 | 178° C |

Tabelle 1 (Fortsetzung)

| Bsp. Nr. | n | R | Het | Schmelzpunkt |
|---|---|---|---|---|
| 55 | 1 | 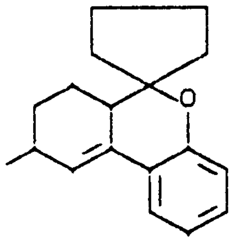 | 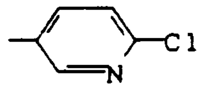 | 155° C |
| 56 | 1 | 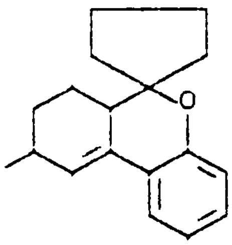 Isomeres | 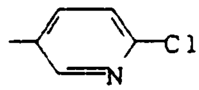 | 158° C |
| 57 | 1 | 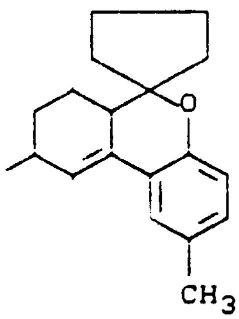 | 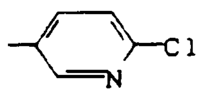 | 140° C |
| 58 | 1 | 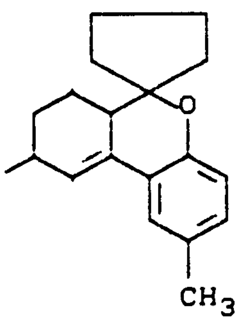 Isomeres | 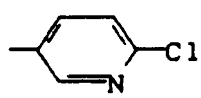 | 170° C |

Tabelle 1 (Fortsetzung)

| Bsp. Nr. | n | R | Het | Schmelzpunkt |
|---|---|---|---|---|
| 59 | 1 | 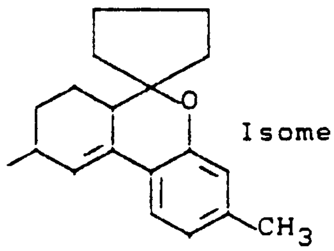 Isomeres | 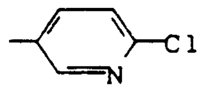 | 115° C |
| 60 | 1 | 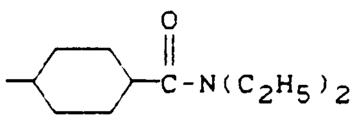 | 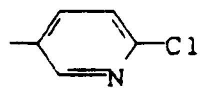 | 149° C |
| 61 | 1 | 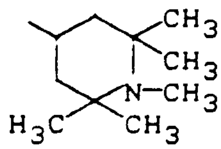 | 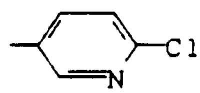 | 164° C |
| 62 | 0 | 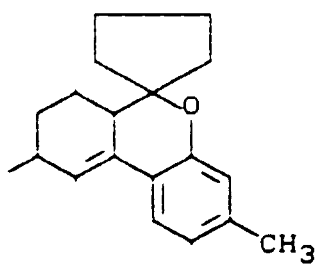 | 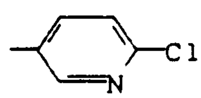 | 170° C |
| 63 | 0 | 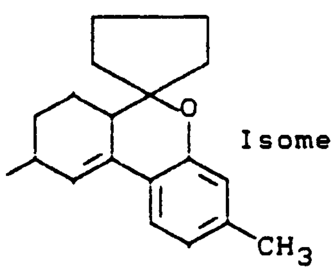 Isomeres | 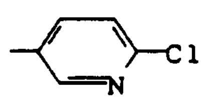 | 82° C |

Tabelle 1 (Fortsetzung)

| Bsp. Nr. | n | R | Het | Schmelzpunkt |
|---|---|---|---|---|
| 64 | 0 | 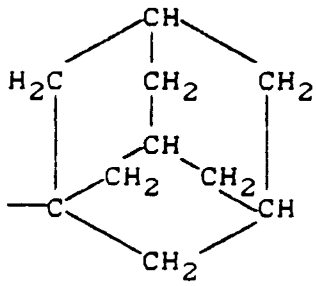 | 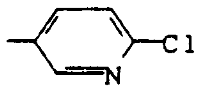 | 183° C |
| 65 | 1 | 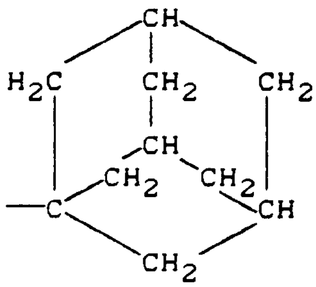 | 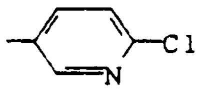 | 164° C |
| 66 | 0 | 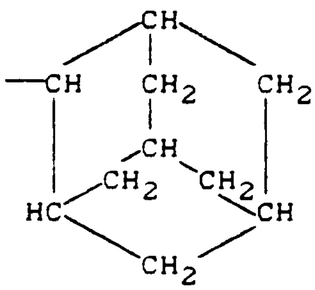 | 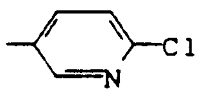 | 164° C |
| 67 | 1 | 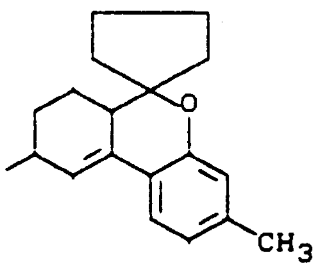 | 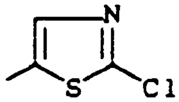 | 130° C (Zers.) |

Tabelle 1 (Fortsetzung)

| Bsp. Nr. | n | R | Het | Schmelzpunkt |
|---|---|---|---|---|
| 68 | 1 | 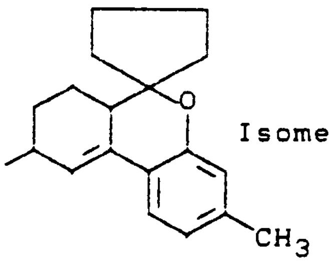 Isomeres | 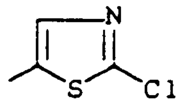 | 174° C (Zers.) |
| 69 | 0 | 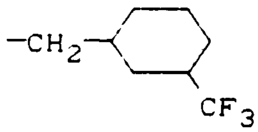 | 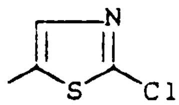 | 125° C |
| 70 | 0 | 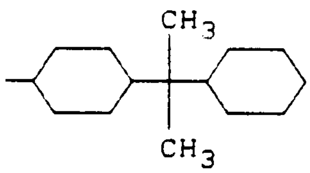 | 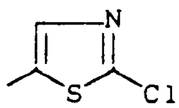 | 155° C |
| 71 | 0 | 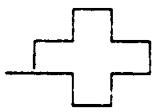 | 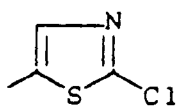 | 167-169° C |
| 72 | 1 | 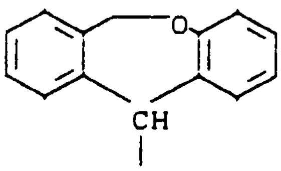 | 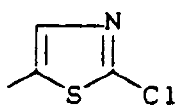 | 203° C |
| 73 | 1 | 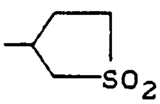 | 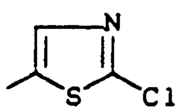 | 3,87;3,91;7,49* |
| 74 | 0 | 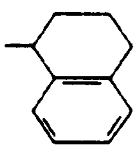 | 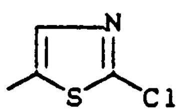 | 103-106° C |

Tabelle 1 (Fortsetzung)

| Bsp. Nr. | n | R | Het | Schmelzpunkt |
|---|---|---|---|---|
| 75 | 1 | 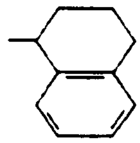 | 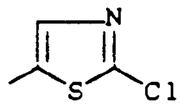 | 169° C (Zers.) |
| 76 | 1 | 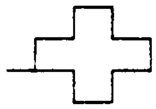 | 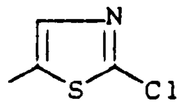 | 185° C (Zers.) |
| 77 | 0 | $-CH_2CH_2CH_2-$N 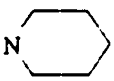 | 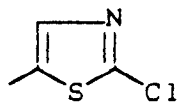 | 119-121° C |
| 78 | 0 | 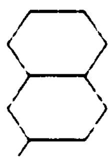 | 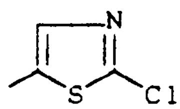 | 148° C (Zers.) |
| 79 | 1 | 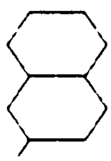 | 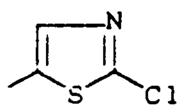 | 133-139° C |
| 80 | 0 | 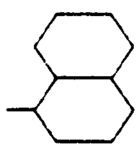 | 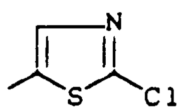 | 148° C (Zers.) |
| 81 | 1 | 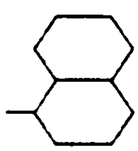 | 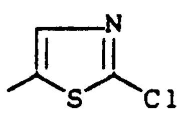 | 150° C (Zers.) |
| 82 | 0 | 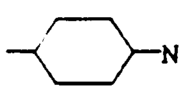 $N(C_2H_5)_2$ | 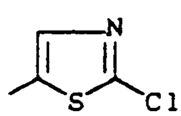 | 122-127° C |

Tabelle 1 (Fortsetzung)

| Bsp. Nr. | n | R | Het | Schmelzpunkt |
|---|---|---|---|---|
| 83 | 1 | 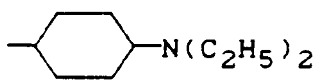 | 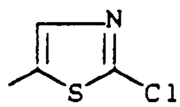 | 157° C (Zers.) |
| 84 | 0 | 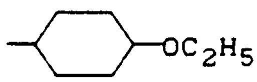 | 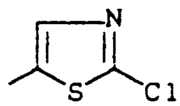 | 83-85° C |
| 85 | 1 | 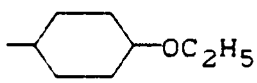 | 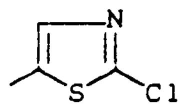 | 127-129° C (Zers.) |
| 86 | 0 | 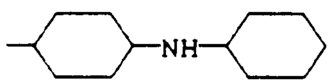 | 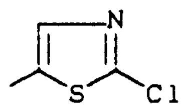 | 136-138° C |
| 87 | 1 | 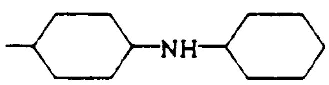 | 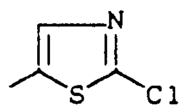 | 161° C (Zers.) |
| 88 | 0 | 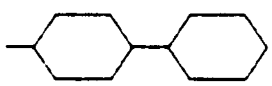 | 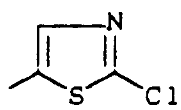 | 210° C (Zers.) |
| 89 | 1 | 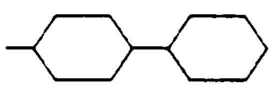 | 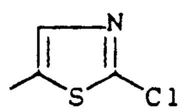 | 180° C (Zers.) |
| 90 | 1 | 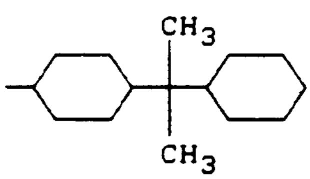 | 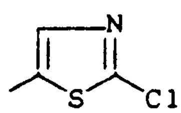 | 155° C (Zers.) |
| 91 | 0 | 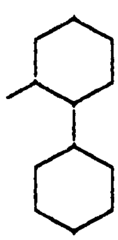 | 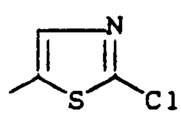 | 74° C |

Tabelle 1 (Fortsetzung)

| Bsp. Nr. | n | R | Het | Schmelzpunkt |
|---|---|---|---|---|
| 92 | 0 | 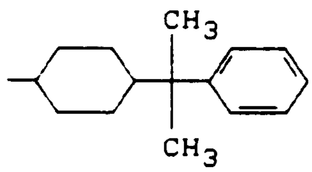 | 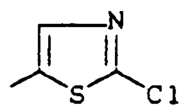 | 170° C (Zers.) |
| 93 | 0 | 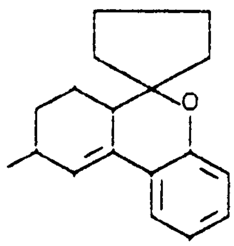 | 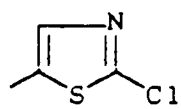 | 105° C (Zers.) |
| 94 | 0 | 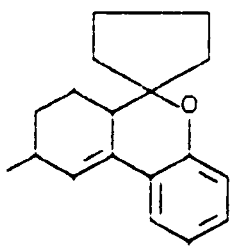 Isomeres | 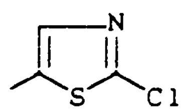 | 107° C (Zers.) |
| 95 | 0 | 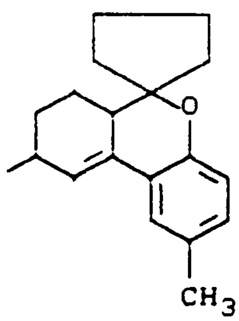 | 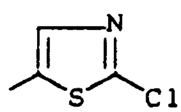 | 184° C (Zers.) |

Tabelle 1 (Fortsetzung)

| Bsp. Nr. | n | R | Het | Schmelzpunkt |
|---|---|---|---|---|
| 96 | 0 | 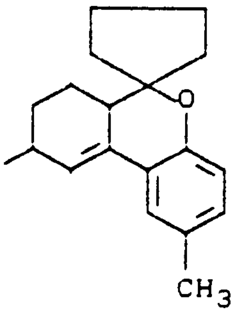 Isomeres | 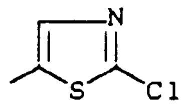 | 117° C (Zers.) |
| 97 | 0 | 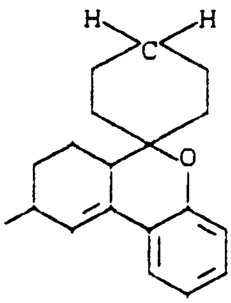 | 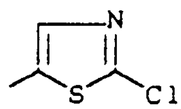 | 150° C (Zers.) |
| 98 | 0 | 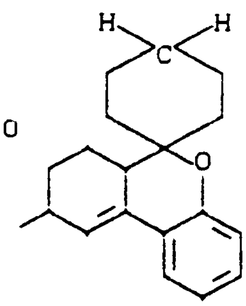 Isomeres | 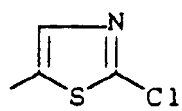 | 178° C (Zers.) |
| 99 | 0 | 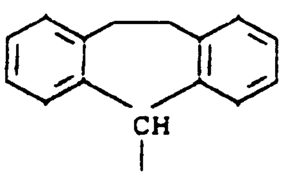 | 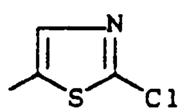 | 213° C (Zers.) |
| 100 | 1 | 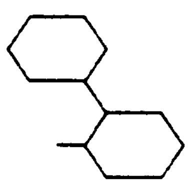 | 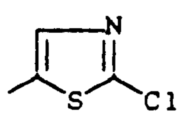 | 87° C |

Tabelle 1 (Fortsetzung)

| Bsp. Nr. | n | R | Het | Schmelzpunkt |
|---|---|---|---|---|
| 101 | 1 | 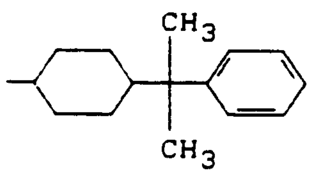 | 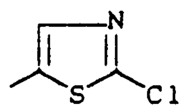 | 172° C (Zers.) |
| 102 | 1 | 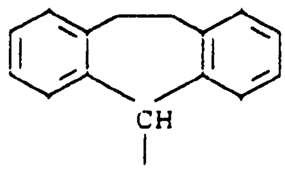 | 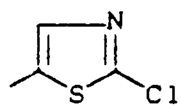 | 224° C |
| 103 | 0 | 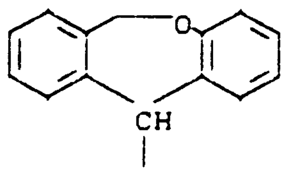 | 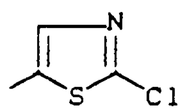 | 151° C (Zers.) |
| 104 | 0 | 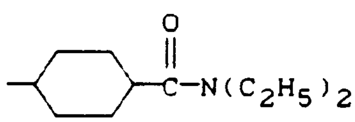 | 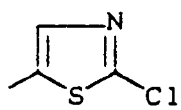 | 96° C (Zers.) |
| 105 | 0 | 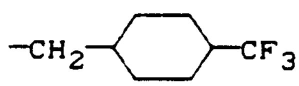 | 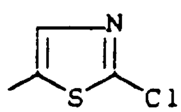 | 79° C |
| 106 | 0 | 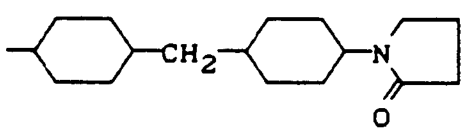 | 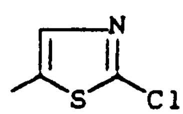 | 151° C (Zers.) |
| 107 | 0 | 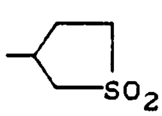 | 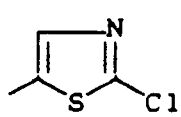 | 196° C |

Tabelle 1 (Fortsetzung)

| Bsp. Nr. | n | R | Het | Schmelzpunkt |
|---|---|---|---|---|
| 108 | 1 | 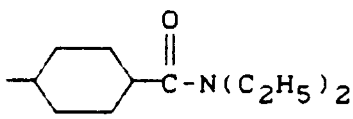 | 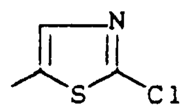 | 143° C (Zers.) |
| 109 | 0 | 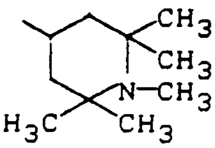 | 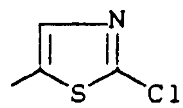 | 167° C (Zers.) |
| 110 | 1 | 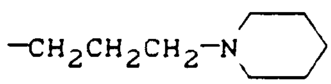 | 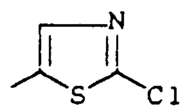 | 122-124° C |
| 111 | 1 | 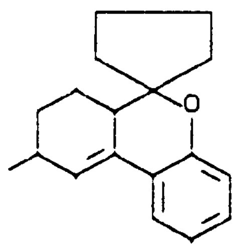 | 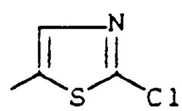 | 124° C (Zers.) |
| 112 | 1 | 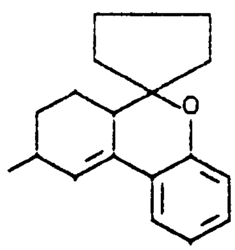 Isomeres | 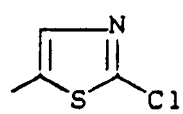 | 124-128° C |

Tabelle 1 (Fortsetzung)

| Bsp. Nr. | n | R | Het | Schmelzpunkt |
|---|---|---|---|---|
| 113 | 1 | 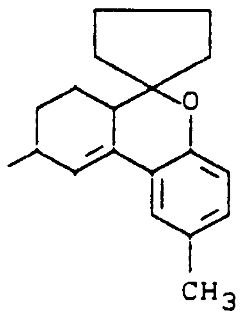 | 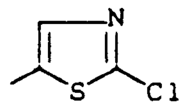 | 120° C (Zers.) |
| 114 | 1 | 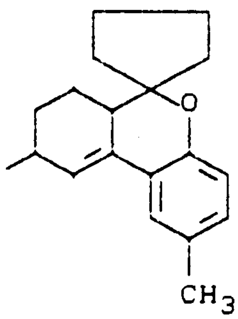 Isomeres | 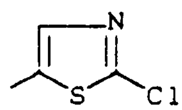 | 165° C (Zers.) |
| 115 | 1 | 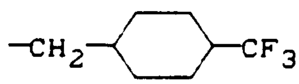 | 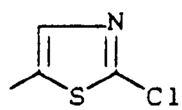 | 126-129° C |
| 116 | 1 | 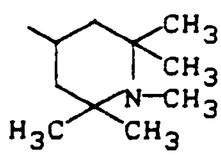 | 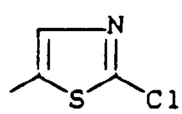 | 169° C (Zers.) |
| 117 | 1 | 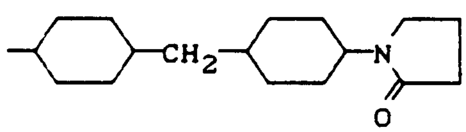 | 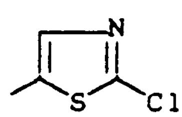 | 153° C (Zers.) |
| 118 | 0 | 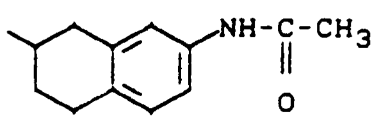 | 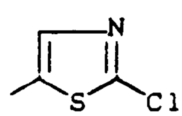 | 132° C |

Tabelle 1 (Fortsetzung)

| Bsp. Nr. | n | R | Het | Schmelzpunkt |
|---|---|---|---|---|
| 119 | 1 | 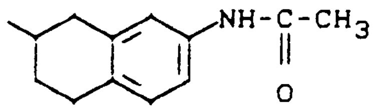 | 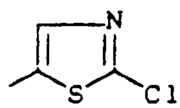 | 148° C |
| 120 | 0 | 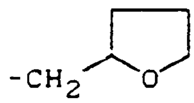 | 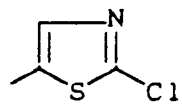 | 150° C |
| 121 | 1 | 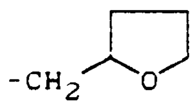 | 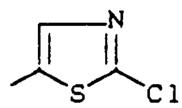 | 161° C |
| 122 | 0 | 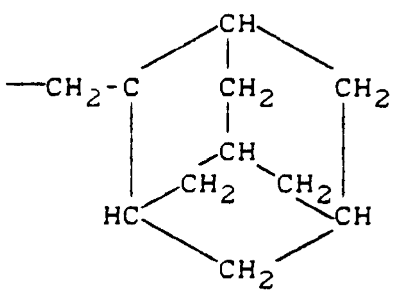 | 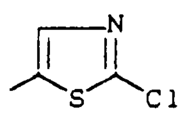 | 214° C |
| 123 | 1 | 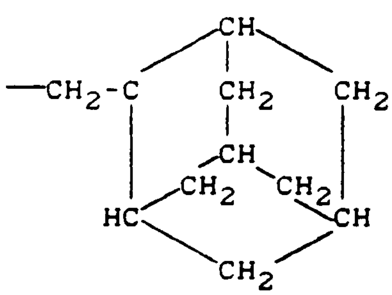 | 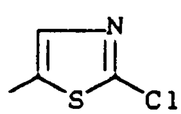 | 196° C |

Tabelle 1 (Fortsetzung)

| Bsp. Nr. | n | R | Het | Schmelzpunkt |
|---|---|---|---|---|
| 124 | 0 | 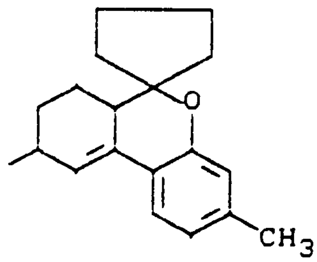 | 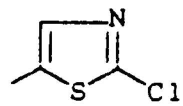 | 154° C |
| 125 | 0 | 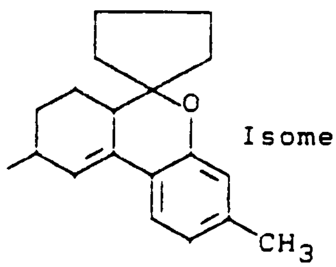 Isomeres | 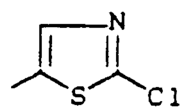 | 89° C |
| 126 | 0 | 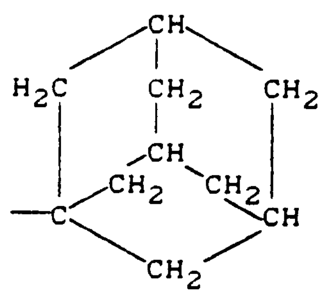 | 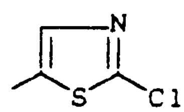 | 210° C |
| 127 | 1 | 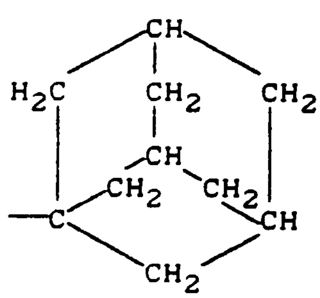 | 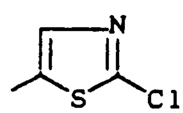 | 203° C |

Tabelle 1 (Fortsetzung)

| Bsp. Nr. | n | R | Het | Schmelzpunkt |
|---|---|---|---|---|
| 128 | 0 | 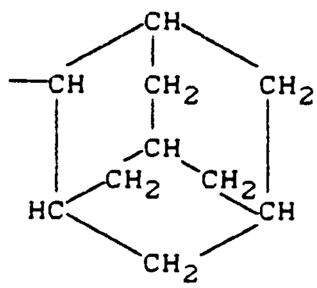 | 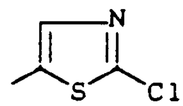 | 208° C |
| 129 | 1 | 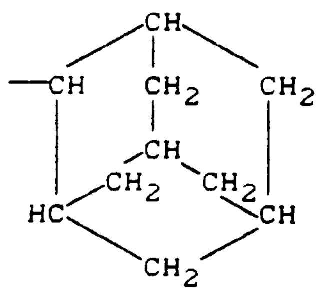 | 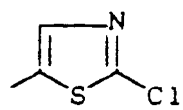 | 154° C |

*) Die $^{1}H$-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. die folgenden Verbindungen der Herstellungsbeispiele 1, 2, 5, 7, 9 bis 40, 70, 71, 74, 77, 78, 79 bis 89, 90 bis 93, 95 bis 97, 100, 101, 103, 104, 106, 108, 109, 111 bis 114, 116 und 117 bei einer beispielhaften Wirkstoffkonzentration von 0,1 % nach 3 Tagen einen Abtötungsgrad von 100 %.

Beispiel B

Aphis-Test (systemische Wirkung)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnentriebe (Vicia faba), die stark von der schwarzen Bohnenlaus (Aphis fabae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. die folgenden Verbindungen der Herstellungsbeispiele 1, 2, 5, 9, 10, 11, 14, 15, 16, 21, 23, 39, 70, 80, 81, 82, 84, 87, 90, 104, 106 und 109 bei einer beispielhaften Konzentration von 0,1 % nach 3 Tagen einen Abtötungsgrad von 100 %.

Beispiel C

Grenzkonzentrations-Test / Bodeninsekten

| | |
|---|---|
| Testinsekt: | Phorbia antiqua-Maden im Boden |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigten z.B. die Verbindungen der Herstellungsbeispiele 5, 71, 77 und 84 bei einer beispielhaften Wirkstoffkonzentration von 20 ppm einen Abtötungsgrad von 100 %.

Beispiel D

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Planzenwurzeln aus dem Boden aufgenommen und in die Blätter transportiert werden.

Für den Nachweis des wurzelsystemischen Effektes werden nach 7 Tagen ausschließlich die Blätter mit den oben genannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z. B. die Verbindungen der Herstellungsbeispiele 1, 2, 5, 7, 11, 13, 14, 15, 16, 17, 21, 23, 32, 70, 71, 77, 86, 87, 89, 93, 94, 95, 100, 110 und 111 bei einer beispielhaften Wirkstoffkonzentration von 20 ppm eine Abtötung von 100 %.

Beispiel E

Grenzkonzentrations-Test / Wurzelsystemische Wirkung

| | |
|---|---|
| Testinsekt: | Phaedon cochleariae-Larven |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Planzenwurzeln aus dem Boden aufgenommen und in die Blätter transportiert werden.

Für den Nachweis des wurzelsystemischen Effektes wegen nach 7 Tagen ausschließlich die Blätter mit den oben genannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z. B. die Verbindungen der Herstellungsbeispiele 2, 11 und 23 bei einer beispielhaften Wirkstoffkonzentration von 20 ppm einen Abtötungsgrad von 100 %.

Beispiel F

Saatgutbehandlungs-Test/Bodeninsekten

| | |
|---|---|
| Testinsekt: | Phorbia antiqua-Maden im Boden |
| Testpflanze: | Albium cepa |
| Lösungsmittel: | 1 Gewichtsteil Aceton |
| Trägermaterial: | Kaolin |

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Zwiebelsaatgut wird mit dieser Wirkstoffzubereitung mit den geforderten Aufwandmengen behandelt. Die Aussaat erfolgt in 0,5-l-Töpfen mit standardisierten Böden bei 20 °C Gewächshaustemperatur.

Nach Auflauf der Zwiebeln werden diese mit Zwiebelfliegeneiern künstlich infiziert.

Die Auswertung erfolgt nach 14 Tagen. Der Wirkungsgrad ist 100 %, wenn alle Zwiebelpflanzen stehenbleiben, 0 %, wenn alle Testpflanzen (wie in der unbehandelten Kontrolle) vernichtet wurden.

Bei diesem Test zeigte z. B. die Verbindung des Herstellungsbeispiels 7 bei einer beispielhaften Menge von 2 g Wirkstoff/kg Saatgut einen Wirkungsgrad von 100 %.

Beispiel G

Saatgutbehandlungs-Test/Wurzelsystemische Wirkung

Testinsekt: Phaedon cochleariae-Käfer
Testpflanze: Brassica oleracea
Lösungsmittel: 1 Gewichtsteil Aceton
Trägermaterial: Kaolin

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Kohl-Saatgut wird mit dieser Wirkstoffzubereitung mit den geforderten Aufwandmengen behandelt. Die Kohl-Aussaat erfolgt in 0,5-l-Töpfen mit standardisierten Böden bei 20 °C Raumtemperatur.

Der Wirkstoff kann so von den Pflanzenwurzeln aus dem Boden aufgenommen und in die Blätter transportiert werden.

Für den Nachweis des wurzelsystemischen Effektes werden nach 14 Tagen ausschließlich die Blätter mit den obengenannten Testtieren besetzt. Nach weiteren 3 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigte z. B. die Verbindung des Herstellungsbeispiels 7 bei einer beispielhaften Menge von 2 g Wirkstoff/kg Saatgut einen Wirkungsgrad von 100 %.

Beispiel H

Saatgutbehandlungs-Test/Wurzelsystemische Wirkung

Testinsekt: Aphis fabae
Testpflanze: Vicia faba
Lösungsmittel: 1 Gewichtsteil Aceton
Trägermaterial: Kaolin

Zur Herstellung einer zweckmäßigen Wirkstoffzubereitung löst man den Wirkstoff in Aceton und vermischt 1 Gewichtsteil Wirkstoff/Aceton mit 5 Gewichtsteilen Kaolin. Das Kohl-Saatgut wird mit dieser Wirkstoffzubereitung mit den geforderten Aufwandmengen behandelt. Die Kohl-Aussaat erfolgt in 0,5-l-Töpfen mit standardisierten Böden bei 20 °C Raumtemperatur.

Der Wirkstoff kann so von den Pflanzenwurzeln aus dem Boden aufgenommen und in die Blätter transportiert werden.

Für den Nachweis des wurzelsystemischen Effektes werden nach 14 Tagen ausschließlich die Blätter mit den obengenannten Testtieren besetzt. Nach weiteren 3 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigte z. B. die Verbindung des Herstellungsbeispiels 5 bei einer beispielhaften Menge von 2 g Wirkstoff/kg Saatgut einen Wirkungsgrad von 100 %.

Beispiel I

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigten z.B. die Verbindungen der Herstellungsbeispiele 1, 2, 5, 6, 7, 9, 10 bis 35, 37, 39, 70, 71, 74 bis 104, 106, 108, 109, 111 bis 114, 116 und 117 bei einer beispielhaften Konzentration von 100 ppm eine Abtötung von 100 %.

Beispiel K

Test mit Blattella germanica

Filterpapierscheiben (Ø 9 cm von Schleicher & Schüll BF) werden in Plastikschalen mit je 2.0 ml der jeweils gewünschten Konzentration der Testsubstanz imprägniert.

Nach Trocknung der Filter werden die Schalen verschlossen und 24 Stunden nach Ansatz mit jeweils 5 mit $CO_2$ betäubten Blattella germanica beschickt.

Dosierungen: 1000, 100, 10 ppm

Die Registrierung der Wirkung erfolgt zu bestimmten Zeiten nach Aufsetzen der Blattella germanica (5 - 360 Minuten bzw. 1 - 7 Tage p. a. nach Aufsetzen der Blattella germanica.

Als Kriterium für den Eintritt der Wirkung gilt das Erreichen des sog. adynamischen Stadiums (keine Lebenszeichen) der Testarthropoden.

100 % Wirkung bedeutet, daß alle Blattella germanica tot sind, 0 % Wirkung bedeutet, daß keine Blattella germanica getötet wurden.

Bei diesem Test zeigten z. B. die folgenden Verbindungen der Herstellungsbeispiele 9, 11, 19, 23, 78, 80, 81, 89, 93, 94, 95, 96, 98, 112 und 113 bei einer beispielhaften Konzentration von 1000 ppm eine Abtötung von 100 %.

Beispiel L

Test mit Sitophilus granarius

Filterpapierscheiben (Ø 9 cm von Schleicher & Schüll BF) werden in Plastikschalen mit je 2.0 ml der jeweils gewünschten Konzentration der Testsubstanz imprägniert.

Nach Trocknung der Filter werden die Schalen verschlossen und 24 Stunden nach Ansatz mit jeweils 10 mit $CO_2$-betäubten Sitophilus granarius beschickt.

Die Registrierung der Wirkung erfolgt zu bestimmten Zeiten nach Aufsetzen der Sitophilus granarius (5 - 360 Minuten bzw. 1 - 7 Tage p. a. nach Aufsetzen der Sitophilius granarius).

Als Kriterium für den Eintritt der Wirkung gilt das Erreichen des sog. adynamischen Stadiums (keine Lebenszeichen) der Testarthropoden.

100 % Wirkung bedeutet, daß alle Sitophilus granarius tot sind, 0 % Wirkung bedeutet, daß keine Sitophilus granarius getötet wurden.

Bei diesem Test zeigten z. B. folgende Verbindungen der Herstellungsbeispiele 1, 10 bis 21, 23 bis 30, 32, 34, 35, 37, 70, 78 bis 101, 103, 104, 108, 109, 111 bis 114, 116 und 117 bei einer beispielhaften Konzentration von 1000 ppm eine Abtötung von 100 %.

## Patentansprüche

**1.** 1,2,3,4-Tetrahydro-5-nitro-pyrimidin-Derivate der Formel (I),

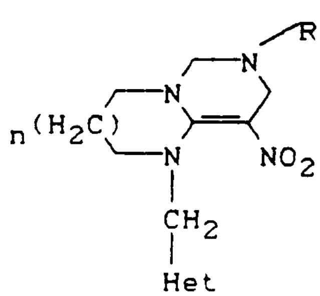

(I)

in welcher

n für die Zahl 0 oder 1 steht,

Het für gegebenenfalls substituiertes Pyridyl oder gegebenenfalls substituiertes Thiazolyl steht

und

R für Monohalogen-tert-Butyl, für unsubstituiertes Cycloalkyl mit 9 bis 18 Kohlenstoffatomen, für Cycloalkyl mit 7 bis 18 Kohlenstoffatomen an welches 1 oder 2 Carbocyclen anelliert sind, für einfach oder mehrfach substituiertes Cyclohexyl welches substituiert ist durch: Alkoxy, Mono- oder Dialkylamino, Cycloalkylamino, Mono- oder Dialkylaminocarbonyl, Cycloalkyl, Cycloalkylalkyl und/oder durch Phenylalkyl, für Cyclohexyl, an welches 1 oder 2 weitere Carbocyclen anelliert sind, für Cycloalkylalkyl mit 3 bis 10 Kohlenstoffatomen im Cycloalkyl- und 1 bis 5 Kohlenstoffatomen im Alkylteil, welches im Cycloalkylteil einfach oder mehrfach substituiert ist, für gegebenenfalls einfach oder mehrfach substituiertes Heterocyclyl mit 2 bis 8 Kohlenstoffatomen und 1 bis 4 Heteroatomen oder Heterogruppierungen, an welches gegebenenfalls 1 oder 2 Carbocyclen anelliert sind, für gegebenenfalls einfach oder mehrfach substituiertes Tetrahydrofurylalkyl mit 1 bis 6 Kohlenstoffatomen im Alkylteil oder für gegebenenfalls einfach oder mehrfach substituiertes Piperidylalkyl mit 1 bis 6 Kohlenstoffatomen im Alkylteil, für Adamantyl, Adamantylmethyl oder für eine der folgenden Gruppierungen (A), (B) und (C), welche gegebenenfalls substituiert sind:

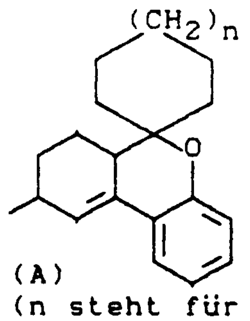

,

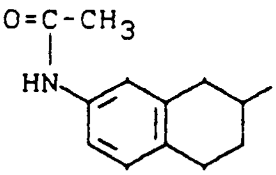

,

(A)
(n steht für 0 oder 1)

(B)

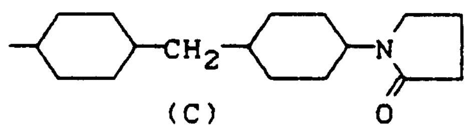

(C)

und deren Säureadditions-Salze.

**2.** Verbindungen der Formel (I) gemäß Anspruch 1, in welcher

n für die Zahlen 0 oder 1 steht,

Het für gegebenenfalls durch Halogen substituiertes Pyridyl oder für gegebenenfalls durch Halogen substituiertes Thiazolyl steht und

R für durch Fluor oder Chlor monosubstituiertes tert-Butyl, für unsubstituiertes Cycloalkyl mit 9 bis 16 Kohlenstoffatomen, für Cycloalkyl mit 7 bis 16 Kohlenstoffatomen, an welches 1 oder 2 6-gliedrige aromatische oder gesättigte Carbocyclen anelliert sind, für einfach bis dreifach substituiertes Cyclohexyl, welches substituiert ist durch: Alkoxy mit 1 bis 4 Kohlenstoffatomen, Mono- oder Dialkylamino mit jeweils 1 bis 4 Kohlenstoffatomen in den einzelnen Alkylteilen, Cycloalkylamino mit 3 bis 7 Kohlenstoffatomen, Mono- oder Dialkylaminocarbonyl mit jeweils 1 bis 4 Kohlenstoffatomen in den einzelnen Alkylteilen, Cycloalkyl mit 3 bis 7 Kohlenstoffatomen, Cycloalkylalkyl mit 3 bis 7 Kohlenstoffatomen im Cycloalkyl- und 1 bis 5 Kohlenstoffatomen im Alkylteil und/oder durch Phenylalkyl mit 1 bis 4 Kohlenstoffatomen im Alkylteil, für Cyclohexyl, an welches 1 oder 2 6-gliedrige aromatische oder gesättigte Carbocyclen anelliert sind, für Cycloalkylalkyl mit 3 bis 7 Kohlenstoffatomen im Cycloalkyl- und 1 bis 4 Kohlenstoffatomen im Alkylteil, welches im Cycloalkylteil einfach bis dreifach durch Alkyl oder Halogenalkyl mit jeweils 1 bis 4 Kohlenstoffatomen und gegebenenfalls 1 bis 9 Fluor-und/oder Chloratomen substituiert ist, für gegebenenfalls einfach oder mehrfach durch Alkyl und/oder Halogenalkyl mit jeweils 1 bis 4 Kohlenstoffatomen und gegebenenfalls 1 bis 9 Fluor-und/oder Chloratomen substituiertes Heterocyclyl mit 3 bis 6 Kohlenstoffatomen und 1 bis 3 Heteroatomen oder Heterogruppierungen aus der Reihe Sauerstoff, Schwefel, Stickstoff, $SO_2$, an welches gegebenenfalls 1 oder 2 6-gliedrige aromatische oder gesättigte Carbocyclen anelliert sind, für gegebenenfalls einfach bis dreifach durch Alkyl

und/oder Halogenalkyl mit jeweils 1 bis 4 Kohlenstoffatomen und gegebenenfalls 1 bis 9 Fluor-und/oder Chloratomen substituiertes Tetrahydrofurylalkyl mit 1 bis 4 Kohlenstoffatomen im Alkylteil oder für gegebenenfalls einfach bis dreifach durch Alkyl und/oder Halogenalkyl mit jeweils 1 bis 4 Kohlenstoffatomen und gegebenenfalls 1 bis 9 Fluor- und/oder Chloratomen substituiertes Piperidylalkyl mit 1 bis 4 Kohlenstoffatomen im Alkylteil oder für eine der folgenden Gruppierungen (A) bis (F) steht, welche gegebenenfalls durch Alkyl mit 1 bis 4 Kohlenstoffatomen substituiert sind:

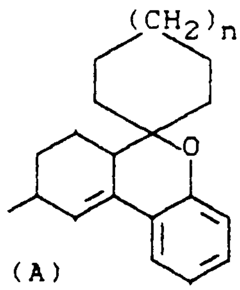

(A)

,

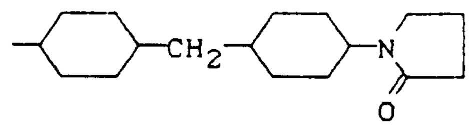

(C)

(n steht für 0 oder 1)

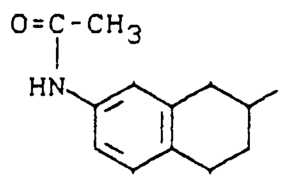

(B)

,

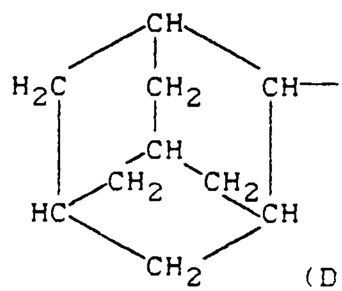

(D)

,

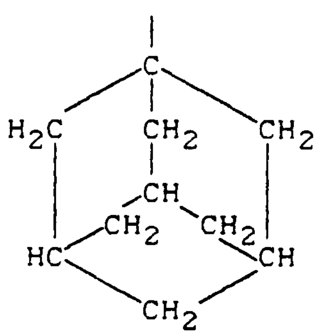

(E)

,

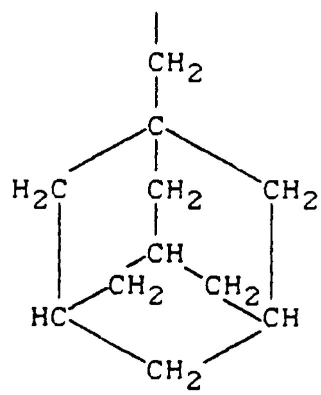

(F).

3. Verbindungen der Formel (I) gemäß Anspruch 1, in welcher

n für die Zahl 0 oder 1 steht,

Het für

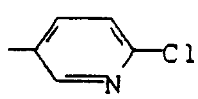

oder

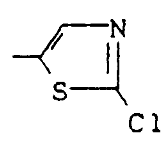

steht und

R für Fluor-tert-Butyl, für unsubstituiertes Cycloalkyl mit 9 bis 12 Kohlenstoffatomen, für Cycloalkyl mit 7 bis 12 Kohlenstoffatomen, an welches 1 oder 2 6-gliedrige aromatische Carbocyclen anelliert sind, für 1- oder 2fach substituiertes Cyclohexyl, welches substituiert ist durch:

Methoxy, Ethoxy, n- oder i-Propoxy, Methylamino, Dimethylamino, Ethylamino, Diethylamino, Ethylmethylamino, n-Propylamino, Di-(n-propyl)amino, Methyl(n-propyl)amino, Ethyl(n-propyl)amino, Isopropylamino, Di(isopropyl)amino, Methylisopropylamino, Ethylisopropylamino, Isopropyl(n-propyl)amino oder Cyclopentylamino, Cyclohexylamino, Methylaminocarbonyl, Dimethylaminocarbonyl, Ethylaminocarbonyl, Diethylaminocarbonyl, Ethylmethylaminocarbonyl, n-Propylaminocarbonyl, Di(n-propyl)aminocarbonyl, Methyl(n-propyl)aminocarbonyl, Ethyl(n-propyl)aminocarbonyl, Isopropylaminocarbonyl, Di(isopropyl)aminocarbonyl, Methylisopropylaminocarbonyl, Ethylisopropylaminocarbonyl, Isopropyl(n-propyl)aminocarbonyl, Cyclopentyl, Cyclohexyl, Cyclopentylmethyl, Cyclopentylethyl, Cyclopentyl-n-propyl, Cyclopentylisopropyl, Cyclohexylmethyl, Cyclohexylethyl, Cyclohexyl-n-propyl, Cyclohexylisopropyl, Phenylmethyl, Phenylethyl, Phenyl-n-propyl und/oder Phenylisopropyl, für Cyclohexyl an welches 1 oder 2 6-gliedrige aromatische oder gesättigte Carbocyclen anelliert sind, für Cyclopentylmethyl, Cyclopentylethyl, Cyclohexylmethyl oder Cyclohexylethyl, wobei diese Reste im Cycloalkylteil 1- oder 2-fach durch Methyl und/oder Trifluormethyl substituiert sind, für gegebenenfalls einfach bis sechsfach durch Methyl und/oder Trifluormethyl substituiertes Heterocyclyl mit 5 bis 12 Kohlenstoffatomen und 1 oder 2 Heteroatomen oder Heterogruppierungen aus der Reihe Sauerstoff, Schwefel, Stickstoff oder $SO_2$, an welches gegebenenfalls 1 oder 2 6-gliedrige aromatische Carbocyclen anelliert sind, für Tetrahydrofurylmethyl oder Tetrahydrofurylethyl, für Piperidylmethyl, Piperidylethyl oder Piperidyl-n-propyl oder für eine der folgenden Gruppierungen (A) bis (F):

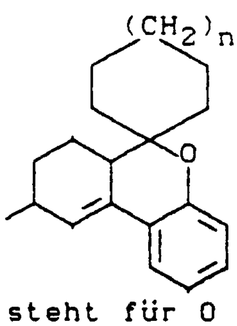

(n steht für 0 oder 1)

(A)

,

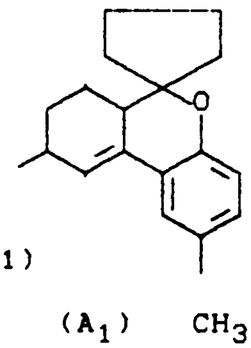

($A_1$)

,

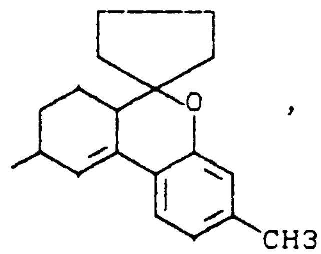

($A_2$)

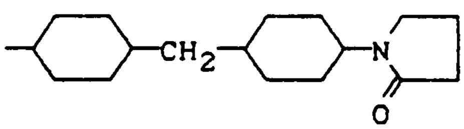

,

(C)

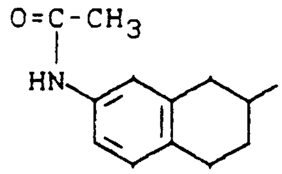 ,

(B)

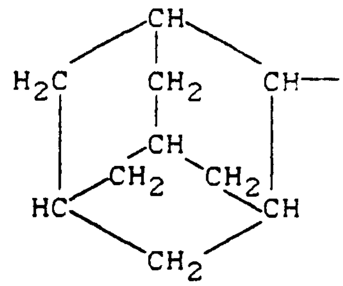 ,

(D)

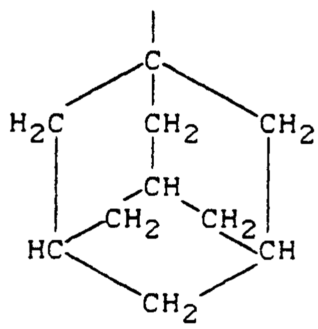 ,

(E)

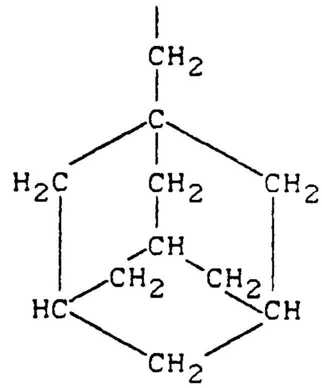

(F).

4. Verfahren zur Herstellung der 1,2,3,4-Tetrahydro-5-nitro-pyrimidin-Derivateder Formel (I),

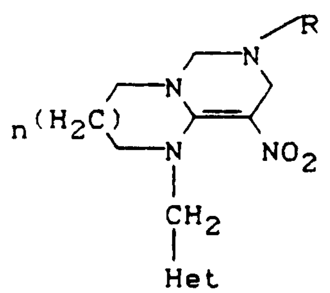

(I)

in welcher

n für die Zahl 0 oder 1 steht,

Het für gegebenenfalls substituiertes Pyridyl oder gegebenenfalls substituiertes Thiazolyl steht und

R für Monohalogen-tert-Butyl, für unsubstituiertes Cycloalkyl mit 9 bis 18 Kohlenstoffatomen, für Cycloalkyl mit 7 bis 18 Kohlenstoffatomen an welches 1 oder 2 Carbocyclen anelliert sind, für einfach oder mehrfach substituiertes Cyclohexyl welches substituiert ist durch: Alkoxy, Mono- oder Dialkylamino, Cycloalkylamino, Mono- oder Dialkylaminocarbonyl, Cycloalkyl, Cycloalkylalkyl und/oder durch Phenylalkyl, für Cyclohexyl, an welches 1 oder 2 weitere Carbocyclen anelliert sind, für Cycloalkylalkyl mit 3 bis 10 Kohlenstoffatomen im Cycloalkyl- und 1 bis 5 Kohlenstoffatomen im Alkylteil, welches im Cycloalkylteil einfach oder mehrfach substituiert ist, für gegebenenfalls einfach oder mehrfach substituiertes Heterocyclyl mit 2 bis 8 Kohlenstoffatomen und 1 bis 4 Heteroatomen oder Heterogruppierungen, an welches gegebenenfalls 1 oder 2 Carbocyclen anelliert sind, für gegebenenfalls einfach oder mehrfach substituiertes Tetrahydrofurylalkyl mit 1 bis 6 Kohlenstoffatomen im Alkylteil oder für gegebenenfalls einfach oder mehrfach substituiertes Piperidylalkyl mit 1 bis 6 Kohlenstoffatomen im Alkylteil, für Adamantyl, Adamantylmethyl oder für eine der folgenden Gruppierungen (A), (B) und (C), welche gegebenenfalls substituiert sind:

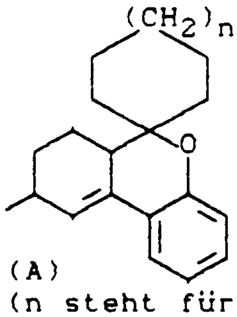

,

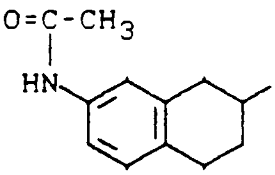

,

(A) (B)

(n steht für 0 oder 1)

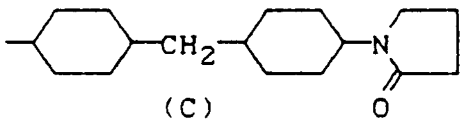

(C)

und deren Säureadditions-Salze,
dadurch gekennzeichnet, daß man Nitromethylen-Derivate der Formel (II)

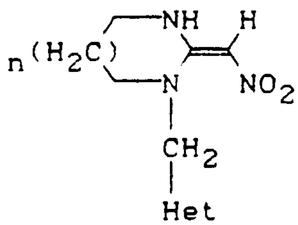

(II)

in welcher
n und Het die oben angegebenen Bedeutungen haben,
mit Aminen der Formel (III),

$R\text{-}NH_2$ (III)

in welcher,
R die oben angegebene Bedeutung hat,
in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen Säuren addiert.

**5.** Schädlingsbekämpfungsmittel, gekennzeichnet durch einen Gehalt an mindestens einer Verbindung der Formel (I), gemäß den Ansprüchen 1 oder 4.

**6.** Verwendung von Verbindungen der Formel (I) gemäß den Ansprüchen 1 oder 4 zur Bekämpfung von Schädlingen.

**7.** Verfahren zur Bekämpfung von Schädlingen, dadurch gekennzeichnet, daß man Verbindungen der Formel (I) gemäß den Ansprüchen 1 oder 4 auf Schädlinge und/oder ihren Lebensraum einwirken läß.

**8.** Verfahren zur Herstellung von Schädlingsbekämpfungsmitteln, dadurch gekennzeichnet, daß man Verbindungen der Formel (I) gemäß den Ansprüchen 1 oder 4 mit Streckmitteln und/oder oberflächenaktiven Mitteln vermischt.

## Claims

**1.** 1,2,3,4-tetrahydro-5-nitro pyrimidine derivatives of the formula (I)

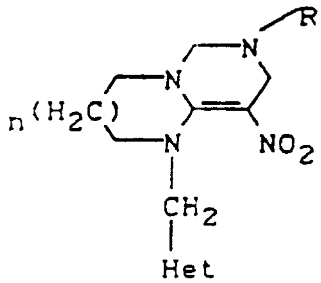

(I)

in which

n represents the number 0 or 1,

Het represents optionally substituted pyridyl or optionally substituted thiazolyl and

R represents monohalogeno-tert-butyl, unsubstituted cycloalkyl having 9 to 18 carbon atoms, cycloalkyl having 7 to 18 carbon atoms to which 1 or 2 carbocycles are fused, monosubstituted or polysubstituted cyclohexyl which is substituted by: alkoxy, mono- or dialkylamino, cycloalkylamino, mono- or dialkylaminocarbonyl, cycloalkyl, cycloalkylalkyl and/or by phenylalkyl, cyclohexyl to which 1 or 2 other carbocycles are fused, cycloalkylalkyl having 3 to 10 carbon atoms in the cycloalkyl and 1 to 5 carbon atoms in the alkyl moiety, which is monosubstituted or polysubstituted in the cycloalkyl moiety, heterocyclyl having 2 to 8 carbon atoms and 1 to 4 heteroatoms or heterogroupings which is optionally monosubstituted or polysubstituted and to which 1 or 2 carbocycles are optionally fused, tetrahydrofurylalkyl having 1 to 6 carbon atoms in the alkyl moiety and which is optionally monosubstituted or polysubstituted or piperidylalkyl having 1 to 6 carbon atoms in the alkyl moiety which is optionally monosubstituted or polysubstituted, adamantyl, adamantylmethyl or one of the following groupings (A), (B) and (C), which are optionally substituted:

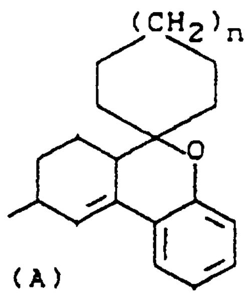

(A) ,

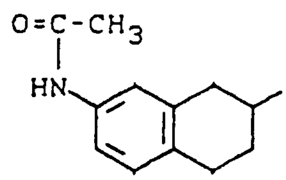

(B) ,

(n represents 0 or 1)

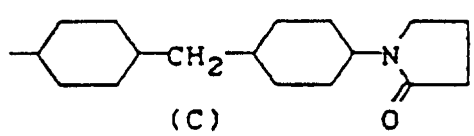

(C)

and their acid addition salts.

**2.** Compounds of the formula (I) according to Claim 1, in which

n represents the numbers 0 or 1,

Het represents pyridyl optionally substituted by halogen or thiazolyl optionally substituted by

halogen and

R represents tert-butyl, monosubstituted by fluorine or chlorine, unsubstituted cycloalkyl having 9 to 16 carbon atoms, cycloalkyl having 7 to 16 carbon atoms, to which 1 or 2 6-membered aromatic or saturated carbocycles are fused, monosubstituted to trisubstituted cyclohexyl which is substituted by: alkoxy having 1 to 4 carbon atoms, mono- or dialkylamino in each case having 1 to 4 carbon atoms in the individual alkyl moieties, cycloalkylamino having 3 to 7 carbon atoms, mono- or dialkylaminocarbonyl in each case having 1 to 4 carbon atoms in the individual alkyl moieties, cycloalkyl alkyl having 3 to 7 carbon atoms, cycloalkylalkyl having 3 to 7 carbon atoms in the cycloalkyl and 1 to 5 carbon atoms in the alkyl moiety and/or by phenylalkyl having 1 to 4 carbon atoms in the alkyl moiety, cyclohexyl, to which 1 or 2 6-membered aromatic or saturated carbocycles are fused, cycloalkylalkyl having 3 to 7 carbon atoms in the cycloalkyl and 1 to 4 carbon atoms in the alkyl moiety, which is monosubstituted to trisubstituted in the cycloalkyl moiety by alkyl or halogenoalkyl in each case having 1 to 4 carbon atoms and optionally 1 to 9 fluorine and/or chlorine atoms, heterocyclyl having 3 to 6 carbon atoms and 1 to 3 heteroatoms or heterogroupings from the series comprising oxygen, sulphur, nitrogen and $SO_2$, which is optionally monosubstituted or polysubstituted by alkyl and/or halogenoalkyl in each case having 1 to 4 carbon atoms and optionally 1 to 9 fluorine and/or chlorine atoms and to which 1 or 2 6-membered aromatic or saturated carbocycles are optionally fused, tetrahydrofurylalkyl having 1 to 4 carbon atoms in the alkyl moiety which is optionally monosubstituted to trisubstituted by alkyl and/or halogenoalkyl in each case having 1 to 4 carbon atoms and optionally 1 to 9 fluorine and/or chlorine atoms or piperidylalkyl having 1 to 4 carbon atoms in the alkyl moiety which is optionally monosubstituted to trisubstituted by alkyl and/or halogenoalkyl in each case having 1 to 4 carbon atoms and optionally 1 to 9 fluorine and/or chlorine atoms or one of the following groupings (A) to (F), which are optionally substituted by alkyl having 1 to 4 carbon atoms:

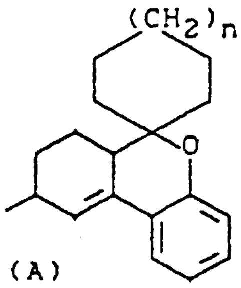

(A)

,

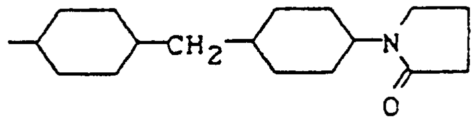

(C)

(n represents 0 or 1)

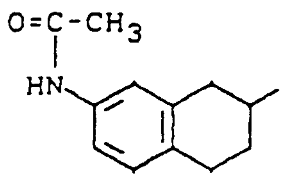

(B)

,

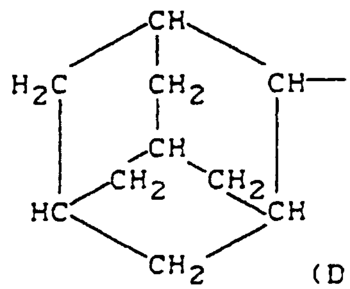

(D)

,

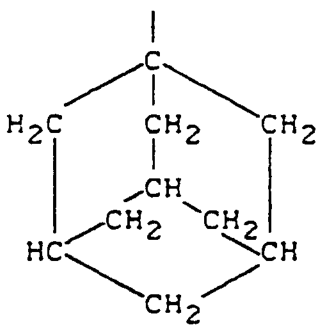

(E)

,

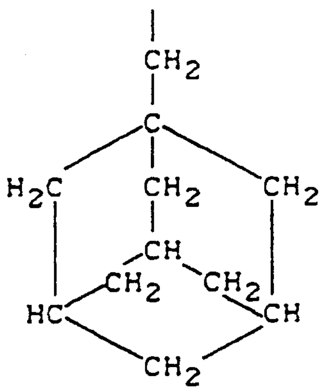

(F).

3. Compounds of the formula (I) according to Claim 1, in which

n represents the number 0 or 1,

Het represents

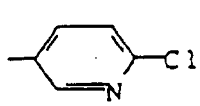 or 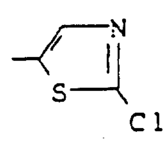

and

R represents fluoro-tert-butyl, unsubstituted cycloalkyl having 9 to 12 carbon atoms, cycloalkyl having 7 to 12 carbon atoms, to which 1 or 2 6-membered aromatic carbocycles are fused, monosubstituted or disubstituted cyclohexyl, which is substituted by: methoxy, ethoxy, n- or i-propoxy, methylamino, dimethylamino, ethylamino, diethylamino, ethylmethylamino, n-propylamino, di-(n-propyl)amino, methyl(n-propyl)amino, ethyl(n-propyl)-amino, isopropylamino, di(isopropyl)amino, methylisopropylamino, ethylisopropylamino, isopropyl(n-propyl)amino or cyclopentylamino, cyclohexylamino, methylaminocarbonyl,

dimethylaminocarbonyl, ethylaminocarbonyl, diethylaminocarbonyl, ethylmethylaminocarbonyl, n-propylaminocarbonyl, di(n-propyl)aminocarbonyl, methyl(n-propyl)aminocarbonyl, ethyl(n-propyl)aminocarbonyl, isopropylaminocarbonyl, di(isopropyl)aminocarbonyl, methylisopropylaminocarbonyl, ethylisopropylaminocarbonyl, isopropyl(n-propyl)-aminocarbonyl, cyclopentyl, cyclohexyl, cyclopentylmethyl, cyclopentylethyl, cyclopentyl-n-propyl, cyclo-pentylisopropyl, cyclohexylmethyl, cyclohexylethyl, cyclohexyl-n-propyl, cyclohexylisopropyl, phenylmethyl, phenylethyl, phenyl-n-propyl and/or phenylisopropyl, cyclohexyl to which 1 or 2 6-membered aromatic or saturated carbocycles are fused, cyclopentylmethyl, cyclopentylethyl, cyclohexylmethyl or cyclohexylethyl, these radicals being monosubstituted or disubstituted in the cycloalkyl moiety by methyl and/or trifluoromethyl, hetero-cyclyl having 5 to 12 carbon atoms and 1 or 2 hetero atoms or heterogroupings from the series comprising oxygen, sulphur, nitrogen and $SO_2$, which is optionally monosubstituted to hexasubstituted by methyl and/or trifluoromethyl and to which 1 or 2 6-membered aromatic carbocycles are optionally fused, tetrahydrofurylmethyl or tetrahydrofurylethyl, piperidylmethyl, piperidylethyl or piperidyl-n-propyl or one of the following groupings (A) to (F):

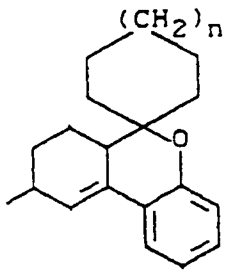 , 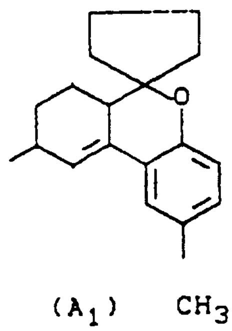 , 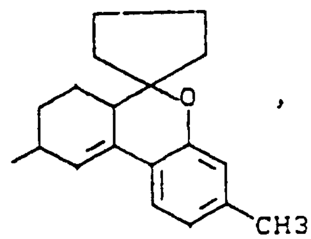 ,

(A) (A$_1$) (A$_2$)

(n represents 0 or 1)

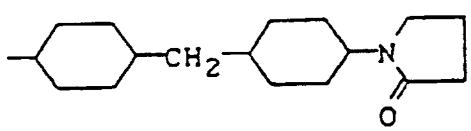 ,

(C)

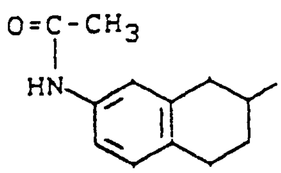

,

(B)

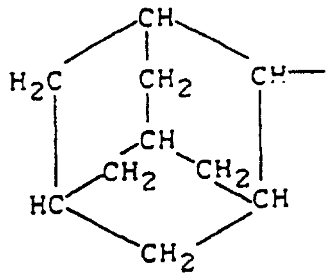

,

(D)

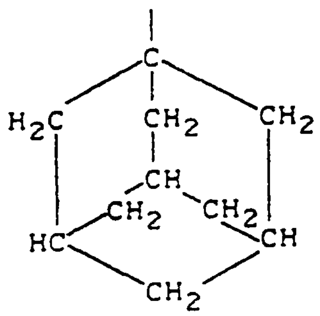

,

(E)

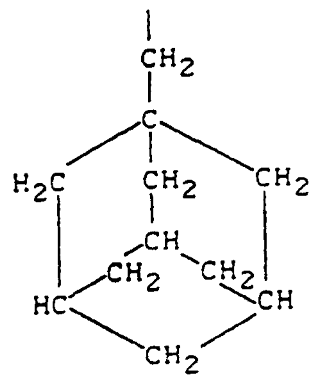

(F).

4. Process for the preparation of the 1,2,3,4-tetra hydro-5-nitropyrimidine derivatives of the formula (I)

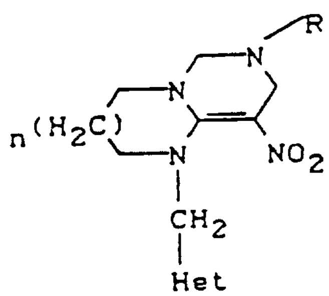

(I)

in which

- n represents the number 0 or 1,
- Het represents optionally substituted pyridyl or optionally substituted thiazolyl and
- R represents monohalogeno-tert-butyl, unsubstituted cycloalkyl having 9 to 18 carbon atoms, cycloalkyl having 7 to 18 carbon atoms to which 1 or 2 carbocycles are fused, monosubstituted or polysubstituted cyclohexyl which is substituted by: alkoxy, mono- or dialkylamino, cycloalkylamino, mono- or dialkylaminocarbonyl, cycloalkyl, cycloalkylalkyl and/or by phenylalkyl, cyclohexyl to which 1 or 2 other carbocycles are fused, cycloalkylalkyl having 3 to 10 carbon atoms in the cycloalkyl and 1 to 5 carbon atoms in the alkyl moiety, which is monosubstituted or polysubstituted in the cycloalkyl moiety, heterocyclyl having 2 to 8 carbon atoms and 1 to 4 heteroatoms or heterogroupings which is optionally monosubstituted or polysubstituted and to which 1 or 2 carbocycles are optionally fused, tetrahydrofurylalkyl having 1 to 6 carbon atoms in the alkyl moiety and which is optionally monosubstituted or polysubstituted or piperidylalkyl having 1 to 6 carbon atoms in the alkyl moiety which is optionally monosubstituted or polysubstituted, adamantyl, adamantylmethyl or one of the following groupings (A), (B) and (C), which are optionally substituted:

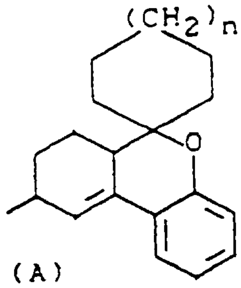

(A) ,

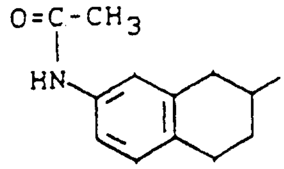

(B) ,

**(n represents 0 or 1)**

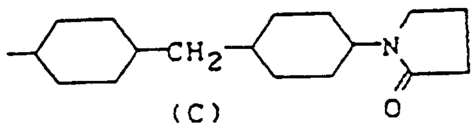

(C)

and their acid addition salts,
characterized in that nitromethylene derivatives of the formula (II)

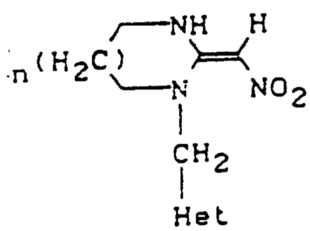

(II)

in which

n and Het have the abovementioned meanings,

are reacted with amines of the formula (III)

$R\text{-}NH_2$ (III)

in which

R has the abovementioned meaning,

in the presence of at least a two-fold molar amount of formaldehyde, if appropriate in the presence of acidic catalysts and if appropriate in the presence of diluents and, if appropriate, acids are adducted to the compounds obtained.

5. Pest-combating agents, characterized in that they contain at least one compound of the formula (I) according to Claims 1 or 4.

6. Use of compounds of the formula (I) according to Claims 1 or 4 for combating pests.

7. Method for combating pests, characterized in that compounds of the formula (I) according to Claims 1 or 4 are allowed to act on pests and/or their environment.

8. Method for the production of pest-combating agents, characterized in that compounds of the formula (I) according to Claims 1 or 4 are mixed with extenders and/or surface-active agents.

## Revendications

**1.** Dérivés de 1,2,3,4-tétrahydro-5-nitropyrimidine de formule (I)

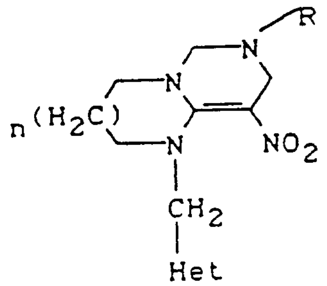

(I)

dans laquelle

n représente le noire 0 ou 1,

Het est un groupe pyridyle éventuellement substitué ou un groupe thiazolyle éventuellement substitué et

R est un groupe monohalogénotertiobutyle, un groupe cycloalkyle non substitué ayant 9 à 18 atomes de carbone, un groupe cycloalkyle ayant 7 à 18 atomes de carbone auquel un ou deux carbocycles sont condensés, un groupe cyclohexyle porteur d'un ou plusieurs substituants, qui est substitué par : un radical alkoxy, mono- ou dialkylamino, cycloalkylamino, mono- ou dialkylaminocarbonyle, cycloalkyle, cycloalkylalkyle et/ou par un radical phénylalkyle, un groupe cyclohexyle auquel un ou deux autres carbocycles sont condensés, un groupe cycloalkylalkyle ayant 3 à 10 atomes de carbone dans la partie cycloalkyle et 1 à 5 atomes de carbone dans la partie alkyle, qui porte dans la partie cycloalkyle un ou plusieurs substituants, un groupe hétérocyclyle portant éventuellement un ou plusieurs substituants et ayant 2 à 8 atomes de carbone et 1 à 4 hétéro-atomes ou groupements hétéro, auquel sont condensés le cas échéant un ou deux carbocycles, un groupe tétrahydrofurylalkyle portant le cas échéant un ou plusieurs substituants et ayant 1 à 6 atomes de carbone dans la partie alkyle ou un groupe pipéridylalkyle portant le cas échéant un ou plusieurs susbstituants et ayant 1 à 6 atomes de carbone dans la partie alkyle, un groupe adamantyle, adamantylméthyle ou l'un des groupements (A), (B) et (C) suivants, qui sont éventuellement substitués

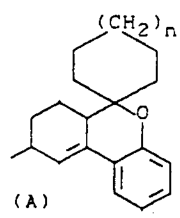

(A) ,

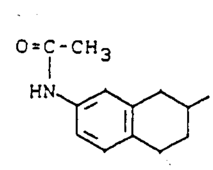

(B) ,

(n a la valeur 0 ou 1)

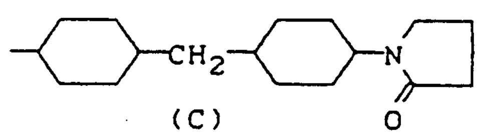

(C)

et leurs sels d'addition d'acides.

**2.** Composés de formule (I) suivant la revendication 1, dans laquelle

n représente le nombre 0 ou 1,

Het est un groupe pyridyle éventuellement substitué par un halogène ou un groupe thiazolyle éventuellement substitué par un halogène et

R est un groupe tertiobutyle monosubstitué par du fluor ou du chlore, un groupe cycloalkyle

non substitué ayant 9 à 16 atomes de carbone, un groupe cycloalkyle de 7 à 16 atomes de carbone, auquel un ou deux carbocycles hexagonaux aromatiques ou saturés sont condensés, un groupe cyclohexyle portant 1 à 3 substituants, qui est substitué par : un radical alkoxy ayant 1 à 4 atomes de carbone, mono- ou dialkylamino ayant chacun 1 à 4 atomes de carbone dans les parties alkyle individuelles, cycloalkylamino ayant 3 à 7 atomes de carbone, mono- ou dialkylaminocarbonyle ayant chacun 1 à 4 atomes de carbone dans les parties alkyle individuelles, cycloalkyle de 3 à 7 atomes de carbone, cycloalkylalkyle de 3 à 7 atomes de carbone dans la partie cycloalkyle et 1 à 5 atomes de carbone dans la partie alkyle et/ou par un radical phénylalkyle ayant 1 à 4 atomes de carbone dans la partie alkyle, un groupe cyclohexyle auquel un ou deux carbocycles hexagonaux aromatiques ou saturés sont condensés, un groupe cycloalkylalkyle de 3 à 7 atomes de carbone dans la partie cycloalkyle et 1 à 4 atomes de carbone dans la partie alkyle, qui est substitué dans la partie cycloalkyle une à trois fois par un radical alkyle ou halogénalkyle ayant chacun 1 à 4 atomes de carbone et le cas échéant 1 à 9 atomes de fluor et/ou de chlore, un groupe hétérocyclyle ayant 3 à 6 atomes de carbone et 1 à 3 hétéro-atomes ou groupements hétéro de la série oxygène, soufre, azote, $SO_2$, substitué le cas échéant une ou plusieurs fois par un radical alkyle et/ou un radical halogénalkyle ayant chacun 1 à 4 atomes de carbone et le cas échéant 1 à 9 atomes de fluor et/ou de chlore, auquel sont condensés le cas échéant 1 ou 2 carbocycles aromatiques ou saturés hexagonaux, un groupe tétrahydrofurylalkyle ayant 1 à 4 atomes de carbone dans la partie alkyle éventuellement substitué une à trois fois par un radical alkyle et/ou un radical halogénalkyle ayant chacun 1 à 4 atomes de carbone et le cas échéant 1 à 9 atomes de fluor et/ou de chlore ou un groupe pipéridylalkyle ayant 1 à 4 atomes de carbone dans la partie alkyle, substitué le cas échéant une à trois fois par un radical alkyle et/ou halogénalkyle ayant chacun 1 à 4 atomes de carbone et le cas échéant 1 à 9 atomes de fluor et/ou de chlore ou l'un des groupements (A) à (F) suivants qui sont substitués le cas échéant par un radical alkyle ayant 1 à 4 atomes de carbone :

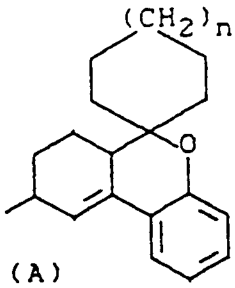

(A)

,

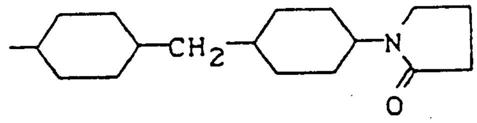

(C)

(n a la valeur 0 ou 1)

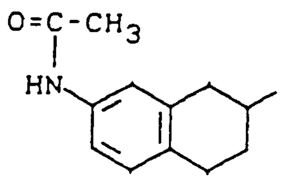

(B)

,

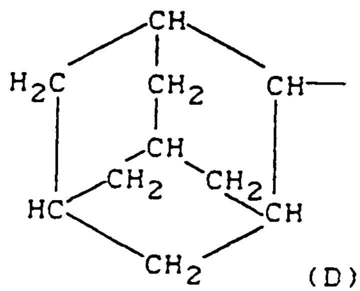

(D)

,

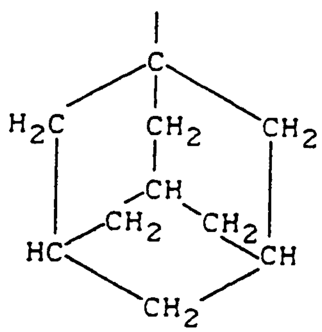

(E)

,

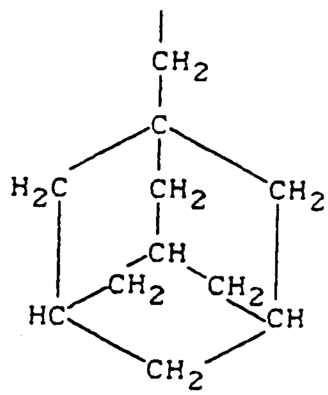

(F).

3. Composés de formule (I) suivant la revendication 1, dans laquelle

n représente le nombre 0 ou 1,

Het est un groupe

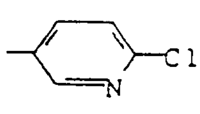

ou

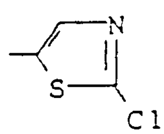

et

R est un groupe fluorotertiobutyle, un groupe cycloalkyle non substitué ayant 9 à 12 atomes de carbone, un groupe cycloalkyle ayant 7 à 12 atomes de carbone auquel sont condensés un ou deux carbocycles aromatiques hexagonaux, un groupe cyclohexyle portant un ou deux substituants, qui est substitué par : un radical méthoxy, éthoxy, n-propoxy, isopropoxy, méthylamino, diméthylamino, éthylamino, diéthylamino, éthylméthylamino, n-propylamino, di(n-propyl)amino, méthyl(n-propyl)amino, éthyl(n-propyl)amino, isopropylamino, di-(isopropyl)amino, méthylisopropylamino, éthylisopropylamino, isopropyl(n-propyl)amino ou

cyclopentylamino, cyclohexylamino, méthylaminocarbonyle, diméthylaminocarbonyle, éthylaminocarbonyle, diéthylaminocarbonyle, éthylméthylaminocarbonyle, n-propylaminocarbonyle, di(n-propyl)aminocarbonyle, méthyl(n-propyl)aminocarbonyle, éthyl(n-propyl)aminocarbonyle, isopropylaminocarbonyle, di(isopropyl)aminocarbonyle, méthylisopropylaminocarbonyle, éthylisopropylaminocarbonyle, isopropyl(n-propyl)aminocarbonyle, cyclopentyle, cyclohexyle, cyclopentylméthyle, cyclopentyléthyle, cyclopentyl-n-propyle, cyclopentylisopropyle, cyclohexylméthyle, cyclohexyléthyle, cyclohexyl-n-propyle, cyclohexylisopropyle, phénylméthyle, phényléthyle, phényl-n-propyle et/ou phénylisopropyle, un groupe cyclohexyle auquel sont condensés un ou deux carbocycles aromatiques ou saturés hexagonaux, un reste cyclopentylméthyle, cyclopentyléthyle, cyclohexylméthyle ou cyclohexyléthyle, ces restes étant substitués dans la partie cycloalkyle une ou deux fois par un radical méthyle et/ou trifluorométhyle, un groupe hétérocyclyle portant le cas échéant 1 à 6 substituants méthyle et/ou trifluorométhyle et ayant 5 à 12 atomes de carbone et 1 ou 2 hétéroatomes ou groupements hétéro de la série oxygène, soufre, azote ou $SO_2$, auquel sont condensés le cas échéant 1 ou 2 carbocycles aromatiques hexagonaux, un groupe tétrahydrofurylméthyle ou tétrahydrofuryléthyle, un groupe pipéridylméthyle, pipéridyléthyle ou pipéridyln-propyle ou l'un des groupements (A) à (F) suivants :

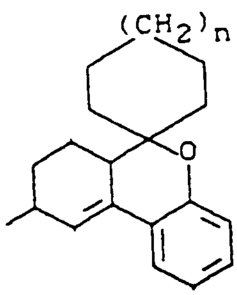

(n a la valeur 0 ou 1)

(A)

,

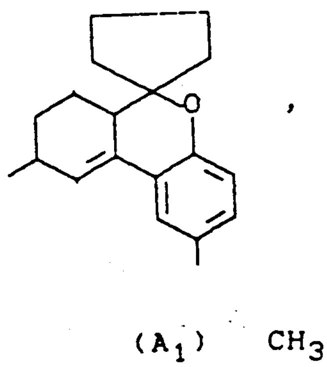

($A_1$)

,

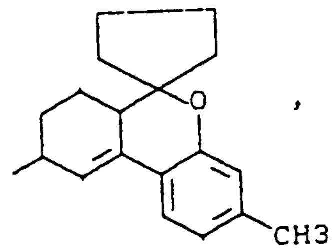

($A_2$)

,

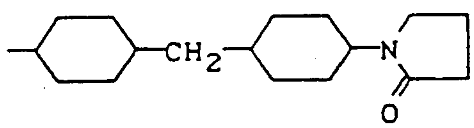

,

(C)

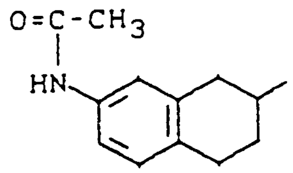

,

(B)

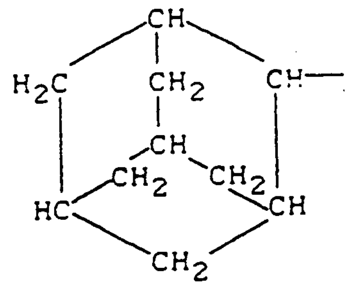

,

(D)

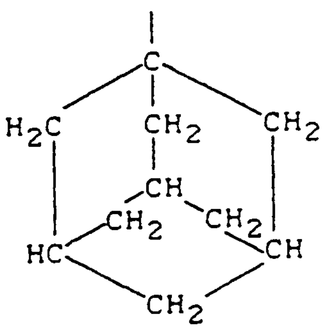

,

(E)

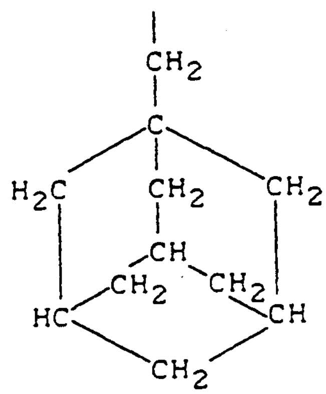

(F).

**4.** Procédé de production des dérivés de 1,2,3,4-tétrahydro-5-nitropyrimidine de formule (I)

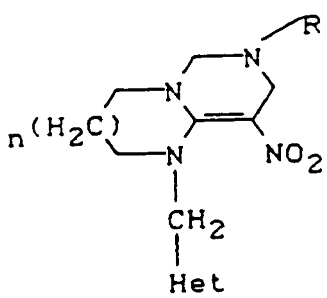

(I)

dans laquelle

- n représente le nombre 0 ou 1,
- Het est un groupe pyridyle éventuellement substitué ou un groupe thiazolyle éventuellement substitué et
- R est un groupe monohalogénotertiobutyle, un groupe cycloalkyle non substitué ayant 9 à 18 atomes de carbone, un groupe cycloalkyle ayant 7 à 18 atomes de carbone auquel un ou deux carbocycles sont condensés, un groupe cyclohexyle porteur d'un ou plusieurs substituants, qui est substitué par : un radical alkoxy, mono- ou dialkylamino, cycloalkylamino, mono- ou dialkylaminocarbonyle, cycloalkyle, cycloalkylalkyle et/ou par un radical phénylalkyle, un groupe cyclohexyle auquel un ou deux autres carbocycles sont condensés, un groupe cycloalkylalkyle ayant 3 à 10 atomes de carbone dans la partie cycloalkyle et 1 à 5 atomes de carbone dans la partie alkyle, qui porte dans la partie cycloalkyle un ou plusieurs substituants, un groupe hétérocyclyle portant éventuellement un ou plusieurs substituants et ayant 2 à 8 atomes de carbone et 1 à 4 hétéro-atomes ou groupements hétéro, auquel sont condensés le cas échéant un ou deux carbocycles, un groupe tétrahydrofurylalkyle portant le cas échéant un ou plusieurs substituants et ayant 1 à 6 atomes de carbone dans la partie alkyle ou un groupe pipéridylalkyle portant le cas échéant un ou plusieurs susbstituants et ayant 1 à 6 atomes de carbone dans la partie alkyle, un groupe

adamantyle, adamantylméthyle ou l'un des groupements (A), (B) et (C) suivants, qui sont éventuellement substitués :

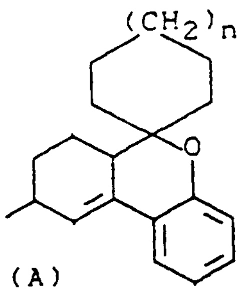

(A) ,

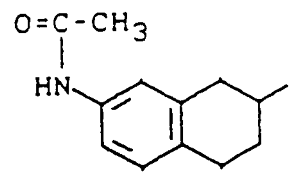

(B) ,

(n a la valeur 0 ou 1)

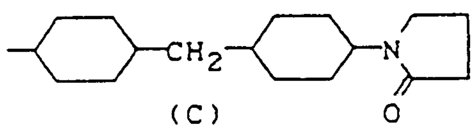

(C)

et de leurs sels d'addition d'acides,

caractérisé en ce qu'on fait réagir des dérivés nitrométhyléniques de formule (II)

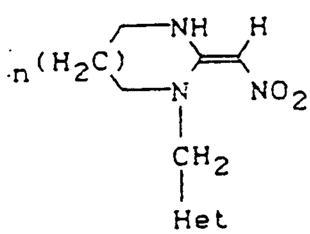

(II)

dans laquelle

n et Het ont les définitions indiquées ci-dessus

avec des amines de formule (III)

$R\text{-}NH_2$ (III)

dans laquelle

R a la définition indiquée ci-dessus,

en présence du double au moins de la quantité molaire de formaldéhyde, le cas échéant en présence de catalyseurs acides et en la présence éventuelle de diluants, et on additionne éventuellement des acides sur les composés obtenus.

**5.** Composition pesticide, caractérisée par une teneur en au moins un composé de formule (I) suivant les revendications 1 ou 4.

**6.** Utilisation de composés de formule (I) suivant les revendications 1 ou 4 pour combattre des parasites.

**7.** Procédé pour combattre des parasites, caractérisé en ce qu'on fait agir sur les parasites et/ou sur leur milieu des composés de formule (I) suivant les revendications 1 à 4.

**8.** Procédé de préparation de compositions pesticides, caractérisé en ce qu'on mélange des composés de formule (I) suivant les revendications 1 ou 4 avec des diluants et/ou avec de agents tensioactifs.